# EUROPEAN PATENT SPECIFICATION

(11) **EP 3 442 436 B1**
(45) Date of publication and mention of the grant of the patent: **13.03.2024**
(21) Application number: 16862793.3
(22) Date of filing: 01.11.2016
(51) Int. Cl.: A61B 17/04, A61F 2/24

(54) **DISTAL ANCHOR APPARATUS FOR MITRAL VALVE REPAIR**
DISTALE ANKERVORRICHTUNG ZUR MITRALKLAPPENREPARATUR
APPAREIL D'ANCRAGE DISTAL POUR RÉPARATION DE VALVULE MITRALE

(30) Priority: 02.11.2015 US 201562249694 P
(43) Date of publication of application: 20.02.2019
(73) Proprietor: University Of Maryland, Baltimore, Baltimore, Maryland 21201 (US); Harpoon Medical, Inc., Baltimore, MD 21201 (US)
(72) Inventor: WILSON, Peter, Killingworth, Connecticut 06419 (US); EPSTEIN, Stephen, Baltimore, Maryland 21224 (US); BOYD, Peter, San Francisco, California 94117 (US); GAMMIE, James, Stevenson, Maryland 21153 (US)
(74) Representative: Eisenführ Speiser
(86) International application number: PCT/US2016/059900
(87) International publication number: WO 2017/079153

(56) References cited:
- WO-A1-2017/066889
- WO-A2-2008/112237
- US-A1- 2005 119 735
- US-A1- 2005 277 966
- US-A1- 2006 207 608
- US-A1- 2008 009 888
- US-A1- 2008 183 194
- US-A1- 2008 183 194
- US-A1- 2011 029 071
- US-A1- 2014 031 926
- US-A1- 2014 031 926
- US-A1- 2015 250 590

## Description

### Background

Some examples described herein relate to apparatus for performing cardiac valve repairs, and more particularly, apparatus for performing minimally invasive mitral or tricuspid valve repairs.

Various disease processes can impair the proper functioning of one or more of the valves of the heart. These disease processes include degenerative processes (e.g., Barlow's Disease, fibroelastic deficiency), inflammatory processes (e.g., Rheumatic Heart Disease), and infectious processes (e.g., endocarditis). Additionally, damage to the ventricle from prior heart attacks (i.e., myocardial infarction secondary to coronary artery disease) or other heart diseases (e.g., cardiomyopathy) can distort the valve's geometry causing it to dysfunction. However, the vast majority of patients undergoing valve surgery, such as mitral valve surgery, suffer from a degenerative disease that causes a malfunction in a leaflet of the valve, which results in prolapse and regurgitation.

Generally, a heart valve may malfunction in two different ways. One possible malfunction, valve stenosis, occurs when a valve does not open completely and thereby causes an obstruction of blood flow. Typically, stenosis results from buildup of calcified material on the leaflets of the valves causing them to thicken and thereby impairing their ability to fully open and permit adequate forward blood flow.

Another possible malfunction, valve regurgitation, occurs when the leaflets of the valve do not close completely thereby causing blood to leak back into the prior chamber. There are three mechanisms by which a valve becomes regurgitant or incompetent; they include Carpentier's type I, type II and type III malfunctions. A Carpentier type I malfunction involves the dilation of the annulus such that the area of the valve orifice increases. The otherwise normally functioning leaflets do not have enough surface area to cover the enlarged orifice and fail to form a tight seal (i.e., do not coapt properly) causing regurgitation. Included in a type I mechanism malfunction are perforations of the valve leaflets, as in endocarditis. A Carpentier's type II malfunction involves prolapse of a segment of one or both leaflets above the plane of the annulus. This is the most common cause of mitral regurgitation, and is often caused by the stretching or rupturing of chordae tendineae normally connected to the leaflet. A Carpentier's type III malfunction involves restriction of the motion of one or more leaflets such that the leaflets are abnormally constrained below the level of the plane of the annulus. Leaflet restriction can be caused by rheumatic disease (Illa) or dilation of the ventricle (IIIb).

Mitral valve disease is the most common valvular heart disorder, with nearly 4 million Americans estimated to have moderate to severe mitral valve regurgitation ("MR"). MR results in a volume overload on the left ventricle which in turn progresses to ventricular dilation, decreased ejection performance, pulmonary hypertension, symptomatic congestive heart failure, atrial fibrillation, right ventricular dysfunction and eventually death. Successful surgical mitral valve repair restores mitral valve competence, abolishes the volume overload on the left ventricle, improves symptom status, prevents adverse left ventricular remodeling and dramatically improves life expectancy, often returning it to that of a normal member of the population.

Malfunctioning valves may either be repaired or replaced. Repair typically involves the preservation and correction of the patient's own valve. Replacement typically involves replacing the patient's malfunctioning valve with a biological or mechanical substitute. Typically, replacement is preferred for stenotic damage sustained by the leaflets because the stenosis is irreversible. The mitral valve and tricuspid valve, on the other hand, are more prone to deformation. Deformation of the leaflets, as described above, prevents the valves from closing properly and allows for regurgitation or back flow from the ventricle into the atrium, which results in valvular insufficiency. Deformations in the structure or shape of the mitral valve or tricuspid valve are often repairable.

In mitral valve regurgitation, repair is preferable to valve replacement. Mitral valve replacement operations have a 2x higher risk of operative mortality (Risk Standardized Mortality 1.65% vs 2.96%), 2x higher risk of stroke per year (1.15% ± 0.1% vs 2.2% ± 0.4%) and a 10x higher risk of infection per year (0.1% vs 1.0%). Patients who receive a quality mitral valve repair operation do not require anticoagulation and rarely require reoperation. This is in stark contrast to mechanical valve replacement which mandates lifelong anticoagulation and bioprosthetic valve replacement with the eventual certainty of prosthetic valve dysfunction and reoperation. Compared to mitral valve replacement, mitral valve repair results in improved left ventricular function and has superior long term survival. Therefore, an improperly functioning mitral valve or tricuspid valve is ideally repaired, rather than replaced. However, because of the complex and technical demands of the repair procedures, the mitral valve is still replaced in approximately one third of all mitral valve operations performed in the United States.

Studies suggest that Carpentier type II malfunction, often referred to as "Degenerative," "Primary" or "Organic" MR, accounts for as much as 60% of MR. Resectional mitral valve repair techniques, initially described by Dr. Carpentier, involve cutting out (resecting) a section of the prolapsed leaflet tissue, stitching the remaining tissue together and implanting an annuloplasty ring around the annulus. More recently many surgeons have moved to a "non-resectional" repair technique where artificial chordae tendineae ("neochords") made of ePTFE suture, or another suitable material, are placed in the prolapsed leaflet and secured to the heart in the left ventricle, normally to the papillary muscle. Because the native leaflet tissue is maintained in non-resectional repairs, they often result in a larger surface of coaptation between the posterior and anterior mitral valve leaflets, but properly sizing the neochords on a flaccid heart can be very challenging, especially for the low volume mitral valve surgeon.

Carpentier type I malfunction, sometimes referred to as "Secondary" or "Functional" MR, is associated with heart failure and affects between 1.6 and 2.8 million people in the United States alone. Studies have shown that mortality doubles in patients with untreated mitral valve regurgitation after myocardial infarction. Unfortunately, there is no gold standard surgical treatment paradigm for functional MR and most functional MR patients are not referred for surgical intervention due to the significant morbidity, risk of complications and prolonged disability associated with cardiac surgery. Surgeons use a variety of approaches ranging from valve replacement to insertion of an undersized mitral valve annuloplasty ring for patients suffering from functional MR and the long term efficacy is still unclear. Dr. Alfieri has demonstrated the benefit of securing the midpoint of both leaflets together creating a double orifice valve in patients with MR known as an "Edge-to-Edge" repair or an Alfieri procedure. The ability to combine a neochordal repair with an edge-to-edge repair in degenerative MR patients with a dilated annulus and who do not receive an annuloplasty ring because the repair is done in a minimally-invasive, off-pump procedure, has particular promise.

Regardless of whether a replacement or repair procedure is being performed, conventional approaches for replacing or repairing cardiac valves are typically invasive open-heart surgical procedures, such as sternotomy or thoracotomy, which require opening up of the thoracic cavity so as to gain access to the heart. Once the chest has been opened, the heart is bypassed and stopped. Cardiopulmonary bypass is typically established by inserting cannulae into the superior and inferior vena cavae (for venous drainage) and the ascending aorta (for arterial perfusion), and connecting the cannulae to a heart-lung machine, which functions to oxygenate the venous blood and pump it into the arterial circulation, thereby bypassing the heart. Once cardiopulmonary bypass has been achieved, cardiac standstill is established by clamping the aorta and delivering a "cardioplegia" solution into the aortic root and then into the coronary circulation, which stops the heart from beating. Once cardiac standstill has been achieved, the surgical procedure may be performed. These procedures, however, adversely affect almost all of the organ systems of the body and may lead to complications, such as strokes, myocardial "stunning" or damage, respiratory failure, kidney failure, bleeding, generalized inflammation, and death. The risk of these complications is directly related to the amount of time the heart is stopped ("cross-clamp time") and the amount of time the subject is on the heart-lung machine ("pump time").
Reference is made to PCT International Applications published as WO 2017/066889 and WO 2008/112237, and U.S. Patent Application Publication No. 2014/0031926.

Thus there is a significant need to perform mitral valve repairs using less invasive procedures while the heart is still beating. Accordingly, there is a continuing need for new procedures and devices for performing cardiac valve repairs, such as mitral valve repair, which are less invasive, do not require cardiac arrest, and are less labor-intensive and technically challenging.

### Summary

Apparatus for performing a minimally-invasive procedure to repair a cardiac valve are described herein. Devices to deliver a distal anchor within the atrium of the heart are described herein. The present invention is defined in the appended claims.

### Brief Description of the Drawings

FIG. 1 is a cut-away anterior view of a heart, showing the internal chambers, valves and adjacent structures.
FIG. 2A is a top perspective view of a healthy mitral valve with the mitral leaflets closed.
FIG. 2B is a top perspective view of a dysfunctional mitral valve with a visible gap between the mitral leaflets.
FIG. 2C is a cross-sectional view of a heart illustrating a mitral valve prolapsed into the left atrium.
FIG. 2D is an enlarged view of the prolapsed mitral valve of Fig. 2C.
FIG. 3 is a cross-sectional view of a heart showing the left atrium, right atrium, left ventricle, right ventricle and the apex region.
FIG. 4 is a schematic illustration of a delivery device, according to an example useful for understanding the present invention, shown inserted into a portion of a heart.
FIG. 5 is a schematic illustration of two anchor-tether apparatus shown implanted within a heart, according to an example not forming part of the present invention.
FIG. 6 is a side view of a distal anchor according to an example not forming part of the present invention, shown in a first delivery configuration.
FIG. 7 is a side view of the distal anchor of FIG. 6 shown in a second deployed configuration.
FIGS. 8A-8C illustrate the transition of the distal anchor of FIGS. 6 and 7 from the first configuration to the second configuration.
FIG. 9 is a schematic illustration of the distal anchor of FIGS. 6 and 7 shown in the first delivery configuration.
FIGS. 10A-10E illustrate in sequence the formation of the distal anchor of FIGS. 6 and 7 about an exterior of a distal end portion of a delivery device, shown in the first delivery configuration.
FIGS. 11A-11H illustrate an example method of forming the distal anchor of FIGS. 6 and 7 about an exterior of a needle, shown in the first delivery configuration.
FIG. 12 is a side view of a distal anchor according to another example not forming part of the present invention, shown in a first delivery configuration.
FIG. 13 is a side view of the distal anchor of FIG. 12 shown in a second deployed configuration.
FIG. 14 is a perspective view of a distal anchor according to another example not forming part of the present invention, shown in a first delivery configuration.
FIG. 15A is a side view of a distal anchor according to another example not forming part of the present invention, shown in a first delivery configuration.
FIG. 15B is a side view of the distal anchor of FIG. 15A shown in a second delivery configuration.
FIGS. 15C and 15D illustrate a side view and a perspective view, respectively, of the distal anchor of FIG. 15A shown in a deployed configuration.
FIG. 16A is a side view of a distal anchor according to another example not forming part of the present invention, shown in a delivery configuration; FIG. 16B is a side view of the distal anchor of FIG. 16A shown in a partially deployed configuration; and FIG. 16C is a side view of the distal anchor of FIG. 16A in a deployed configuration.
FIG. 17A is a side view of a distal anchor according to another example not forming part of the present invention, shown in a delivery configuration; FIG. 17B is a side view of the distal anchor of FIG. 17A shown in a partially deployed configuration; and FIG. 17C is a side view of the distal anchor of FIG. 17A in a deployed configuration.
FIG. 18A is a side view of a distal anchor according to an embodiment of the present invention, shown in a delivery configuration and disposed within a lumen of a delivery device.
FIG. 18B is illustrates the distal anchor of FIG. 18A in the delivery configuration.
FIG. 18C illustrates the distal anchor of FIG. 18A in a partially deployed configuration.
FIG. 18D illustrate the distal anchor of FIG. 18A in a deployed configuration.
FIGS. 19A and 19B illustrate the distal anchor of FIG. 18A, shown in the deployed configuration.
FIGS. 20A-20C illustrate a distal anchor according to another example not forming part of the present invention, shown in a deployed configuration.
FIG. 21A illustrates a distal anchor according to another example not forming part of the present invention, shown in a delivery configuration.
FIG. 21B illustrates the distal anchor of FIG. 21A, shown with reference to a valve leaflet and in the delivery configuration.
FIG. 21C illustrates in cross-section the distal anchor of FIG. 21A, shown in the delivery configuration.
FIG. 21D illustrates the distal anchor of FIG. 21A, shown with reference to the valve leaflet and in a deployed configuration.
FIG. 21E illustrates in cross-section the distal anchor of FIG. 21A, shown with reference to the valve leaflet and in the deployed configuration.
FIG. 22A and 22B are side views of a distal anchor according to another example not forming part of the present invention, shown in a delivery configuration and a deployed configuration, respectfully.
FIG. 22C is a perspective view of the distal anchor of FIGS. 22A and 22B, shown in the deployed configuration.
FIG. 23A illustrates a distal anchor according to another example not forming part of the present invention, shown in a in a delivery configuration.
FIG. 23B is a schematic of the distal anchor of FIG. 23A, shown in a deployed configuration.

### Detailed Description

Apparatus and methods for performing a non-invasive procedure to repair a cardiac valve, such as a mitral valve or tricuspid valve, are described herein. In some examples not forming part of the present invention, a method for repairing a mitral valve includes inserting a delivery device through an apex region of a heart and extending a distal end of the delivery device to the proximal side of a leaflet of the mitral valve. A piercing portion of the delivery device can be used to form an opening in the leaflet, through which the distal end of the delivery device can be inserted. The delivery device can be used to form or deliver a distal anchor to the distal side of the leaflet. The location of the opening in the leaflet and the placement of the distal anchor can be anywhere in the leaflet from the free edge up to the base of the mitral valve leaflet and even in the mitral-annular curtain or annulus of the valve. The delivery device can then be withdrawn and a tether coupled to the distal anchor can be secured to an outer surface of the heart at the apex region with, for example, a proximal anchor. The combined distal anchor, tether and proximal anchor is also referred to herein as an anchor-tether apparatus. Before the proximal anchor of the anchor-tether apparatus is fixed to the heart, the length of the tether portion can be adjusted so that the distal movement during systole of the prolapsed segment of the prolapsed leaflet to which the tether portion is coupled by the distal anchor is limited by the tether apparatus during systole. Properly adjusting the length of the anchor-tether apparatus while the heart is beating allows the operator to precisely titrate the position of the prolapsed segment of the prolapsed leaflet in real time to prevent the leaflet from extending above the plane of the annulus (prolapsing), but so that the prolapsed segment of the prolapsed leaflet can move distally during systole a sufficient distance to coapt properly with the other leaflet(s). This adjustment can involve shortening or lengthening the tether portion between the distal and proximal anchors of the anchor-tether apparatus. The same procedure can be repeated on the same leaflet to deliver one or more additional anchor-tether apparatuses to the leaflet, and or can be performed on the other leaflet of the mitral valve to deliver one more anchor-tether apparatuses to the other leaflet (or to both of the other leaflets, in the case of a tricuspid valve). In the case of multiple anchor-tether apparatuses, the tether adjustment procedure can be done one at a time or all at once with the goal of maximizing the surface of coaptation between the leaflets, and eliminating MR.

In some examples useful for understanding the present invention, a delivery device is provided to perform the above repair procedure. Such a delivery device can include, for example a distal end portion that includes a piercing portion and a support portion, an elongate member coupled to the distal end portion, and an actuating handle coupled to a proximal end portion of the elongate member. The piercing portion of the distal end portion of the delivery device can be used to form the opening in the leaflet of the mitral valve. The support portion of the distal end portion can be used to deliver or form the distal anchor. The handle can include a tether control device that can be used to hold the tether extending from the distal anchor and secure the tether to the apex region with the proximal anchor.

In some examples not forming part of the present invention, an apparatus includes a needle having a bore and a distal tip suitable for penetrating a heart valve leaflet, a pusher having a distal end, a distal anchor having a distal end, a proximal end, a plurality of radially expandable members between the distal end and the proximal end, and a central bore extending between the distal end and the proximal end, and an artificial chordae having a proximal portion and a distal portion terminating in a stopper. The pusher is disposed within the bore of the needle for movement between a delivery position in which the distal end of the pusher is proximate to the distal tip of the needle and a deployed position in which the distal end of the pusher is distal to the distal tip of the needle. The distal anchor is disposed within the bore of the needle in a delivery configuration in which the radially expandable members are oriented substantially axially and movable between a delivery position in which the distal anchor is disposed between the distal end of the pusher and the distal tip of the needle and a deployed position in which the plurality of radially expandable members are disposed distal to the distal tip of the needle. The artificial chordae is disposed within the bore of the distal anchor with the stopper distal to the distal end of the stopper and the proximal portion proximal to the proximal end of the distal anchor. The distal anchor is reconfigurable from the delivery configuration to a deployed configuration when the distal anchor is in the deployed position in which the radial extensions extend radially outwardly from the artificial chordae a distance sufficient to anchor the artificial chordae to the heart valve leaflet.

According to the present invention, an apparatus includes a needle having a bore and a distal tip suitable for penetrating a heart valve leaflet, a pusher having a distal end and disposed within the bore of the needle for movement between a delivery position and a deployed position, a distal anchor, and an artificial chordae having a proximal portion and a distal portion terminated in a stopper. In the delivery position, the distal end of the pusher is proximate to the distal tip of the needle. In the deployed position, the distal end of the pusher is distal to the distal tip of the needle. The distal anchor has a distal end and proximal end. The distal end of the distal anchor has a stopper receiving portion. The distal anchor has multiple elongate members extending proximally from the stopper receiving portion. Each elongate member has a free proximal that defines the proximal end of the distal anchor. The distal anchor has a central bore extending through the stopper receiving portion and between the elongate members. The distal anchor is disposed within the bore of the needle in a delivery configuration in which the elongate members are oriented substantially axially and movable between a delivery position in which the distal anchor is disposed between the distal end of the pusher and the distal tip of the needle and a deployed position in which the proximal end of the distal anchor is disposed distal to the distal tip of the needle. The artificial chordae is disposed within the bore of the distal anchor with the stopper distal to the stopper receiving portion of the distal anchor and the proximal portion proximal to the proximal end of the distal anchor. The distal anchor is reconfigurable from the delivery configuration to a deployed configuration when the distal anchor is in the deployed position in which the elongate members are disposed with their proximal ends spaced radially outwardly from the artificial chordae a distance sufficient to anchor the artificial chordae to the heart valve leaflet. The distal anchor is reconfigurable from the delivery configuration to the deployed configuration by moving the distal end of the pusher distally towards the distal end of the distal anchor past the free proximal ends of, and between, the elongate members to urge the elongate members radially outwardly.

In some examples not forming part of the present invention, an apparatus includes a needle having a longitudinal axis, a bore and a distal tip suitable for penetrating a heart valve leaflet, a pusher having a distal end and disposed within the bore of the needle for movement between a delivery position and a deployed position, a distal anchor having an elongate body with a first end and an opposite, second end, and an artificial chordae. In the delivery position, the distal end of the pusher is proximate to the distal tip of the needle. In the deployed position, the distal end of the pusher is distal to the distal tip of the needle. The distal anchor is disposed within the bore of the needle in a delivery configuration in which the elongate body is oriented parallel to the bore of the needle and movable between a delivery position in which the distal anchor is disposed between the distal end of the pusher and the distal tip of the needle and a deployed position in which the distal anchor is disposed distal to the distal tip of the needle. The artificial chordae has a proximal portion and a distal end coupled to the body of the distal anchor between the first end and the second end of the distal anchor. The distal anchor is reconfigurable from the delivery configuration to a deployed configuration when the distal anchor is in the deployed position in which the body of the distal anchor is oriented transvers to the longitudinal axis of the needs sufficient to anchor the artificial chordae to the heart valve leaflet.

In some examples not forming part of the present invention, an apparatus includes a needle having a longitudinal axis, a bore and a distal tip suitable for penetrating a heart valve leaflet, a pusher having a distal end and disposed within the bore of the needle for movement between a delivery position and a deployed position, an umbrella anchor having a closed distal end and an open proximal end, and an artificial chordae having a proximal portion and a distal end coupled to the distal end of the umbrella anchor. In the delivery position, the distal end of the pusher is proximate to the distal tip of the needle. In the deployed position, the distal end of the pusher is distal to the distal tip of the needle. The umbrella anchor is disposed within the bore of the needle in a delivery configuration and movable between a delivery position in which the umbrella anchor is disposed between the distal end of the pusher and the distal tip of the needle and a deployed position in which the umbrella anchor is disposed distal to the distal tip of the needle. The umbrella anchor is reconfigurable from the delivery configuration to a deployed configuration when the umbrella anchor is in the deployed position in which the open proximal end of the body of the umbrella anchor extends radially outwardly from the artificial chordae a distance sufficient to anchor the artificial chordae to the heart valve leaflet.

In some examples not forming part of the present invention, an apparatus including a needle having a bore and a distal tip suitable for penetrating a heart valve leaflet, a pusher having a distal end and disposed within the bore of the needle for movement between a delivery position and a deployed position, a distal anchor, and an artificial chordae having a proximal portion and a distal end coupled to a distal braided portion. In the delivery position, the distal end of the pusher is proximate to the distal tip of the needle. In the deployed position, the distal end of the pusher is distal to the distal tip of the needle. The distal anchor has a distal end, a distal braided portion proximal to the distal end, and distal collar proximal to the distal braided portion and having a diameter, and proximal braided portion proximal to the distal collar, and a proximal collar at the proximal end of the distal anchor, proximal to the proximal braided portion. The distal anchor is disposed within the bore of the needle in a delivery configuration in which each of the distal braided portion and the proximal braided portion have a diameter no larger than the diameter of the distal collar and having a first axial length and movable between a delivery position in which the distal anchor is disposed between the distal end of the pusher and the distal tip of the needle and a deployed position in which the proximal end of the distal anchor is disposed distal to the distal tip of the needle. The distal anchor is reconfigurable from the delivery configuration to a deployed configuration when the distal anchor is in the deployed position in which each of the distal braided portion and the proximal braided portion has a diameter greater than the diameter of the distal collar and a second axial length less than its respective axial length, the distal anchor configured to be disposed with the distal braided portion on a first side of the heart valve leaflet and the proximal braided portion on a second side of the heart valve leaflet, the diameters of the first braided portion and the second braided portion sufficient to anchor the artificial chordae to the heart valve leaflet.

In some examples not forming part of the present invention, an apparatus includes a needle having a bore, a longitudinal axis, and a distal tip suitable for penetrating a heart valve leaflet, a pusher having a distal end and disposed within the bore of the needle for movement between a delivery position and a deployed position, a distal anchor, and an artificial chordae having a proximal portion and a distal portion terminating in a stopper. In the delivery position, the distal end of the pusher is proximate to the distal tip of the needle. In the deployed position, the distal end of the pusher is distal to the distal tip of the needle. The distal anchor has a tubular body with a distal end, a proximal end, a first elongated segment proximal to the distal end and having a first aperture therethrough, a second elongated segment proximal to the first elongated segment and having a second aperture therethrough, a first hinge coupling the first elongated segment to the second elongated segment for pivotal movement of the first elongated segment relative to the second elongated segment, a third elongated segment proximal to the second elongated segment and having a third aperture therethrough, and a second hinge coupling the second elongated segment to the third elongated segment for pivotal movement of the second elongated segment relative to the third second elongated segment. The distal anchor is disposed within the bore of the needle in a delivery configuration in which the first elongated segment, the second elongated segment, and the third elongated segment are oriented substantially axially and parallel to the longitudinal axis of the needle and movable between a delivery position in which the distal anchor is disposed between the distal end of the pusher and the distal tip of the needle and a deployed position in which the distal anchor is disposed distal to the distal tip of the needle. The artificial chordae is disposed with the stopper adjacent to the first aperture and extends proximally through the first aperture, the second aperture and the third aperture and the proximal portion proximal to the proximal end of the distal anchor. The distal anchor is reconfigurable from the delivery configuration to a deployed configuration when the distal anchor is in the deployed position by relative movement of the stopper towards the distal end of the pusher to cause the first elongated segment to pivot about the first hinge, and the third elongated segment to pivot about the second hinge, relative to the second elongated segment, so that the three elongated segments are disposed approximately parallel, the first hinge is spaced from the longitudinal axis of the needle in a first lateral direction, and the second hinge is spaced from the longitudinal axis of the needle in a second lateral direction.

In some examples not forming part of the present invention, an apparatus includes a needle having a bore, a longitudinal axis, and a distal tip suitable for penetrating a heart valve leaflet, a pusher having a distal end and disposed within the bore of the needle for movement between a delivery position and a deployed position, a distal anchor, and an artificial chordae having a proximal portion and a distal portion terminating in a stopper. In the delivery position, the distal end of the pusher is proximate to the distal tip of the needle. In the deployed position, the distal end of the pusher is distal to the distal tip of the needle. The distal anchor has a tubular body with a distal end, a proximal end, a central bore therethrough, a first elongated segment proximal to the distal end and having a hinge in a central portion thereof, and a second elongated segment proximal to the first elongated segment and having a hinge in a central portion thereof. The distal anchor is disposed within the bore of the needle in a delivery configuration in which the first elongated segment and the second elongated segment are oriented substantially axially and parallel to the longitudinal axis of the needle and movable between a delivery position in which the distal anchor is disposed between the distal end of the pusher and the distal tip of the needle and a deployed position in which the distal anchor is disposed distal to the distal tip of the needle. The artificial chordae is disposed with the stopper adjacent to the distal end of the distal anchor, and extends proximally through the central bore of the distal anchor with the proximal portion proximal to the proximal end of the distal anchor. The distal anchor is reconfigurable from the delivery configuration to a deployed configuration when the distal anchor is in the deployed position by relative movement of the stopper towards the distal end of the pusher to cause the first elongated segment to fold about its hinge and the second elongated segment to fold about its hinge, so that the elongated segments project radially from the longitudinal axis of the needle sufficient to anchor the artificial chordae to the heart valve leaflet.

In some examples not forming part of the present invention, an apparatus includes a needle having a bore, a longitudinal axis, and a distal tip suitable for penetrating a heart valve leaflet, a pusher having a distal end and disposed within the bore of the needle for movement between a delivery position and a deployed position, a distal anchor, and an artificial chordae having a proximal portion and a distal portion coupled to the distal anchor. In the delivery position, the distal end of the pusher is proximate to the distal tip of the needle. In the deployed position, the distal end of the pusher is distal to the distal tip of the needle. The distal anchor has a flexible tubular body with central lumen having a distal end and a proximal end. A distal portion and a proximal portion are separated by a partial circumferential slit communicating with the central lumen. The distal anchor is disposed with the needle extending through the central lumen of the flexible tubular body in a delivery configuration in which the distal portion and the proximal portion are oriented substantially axially and movable between a delivery position in which the distal anchor is disposed between the distal end of the pusher and the distal tip of the needle and a deployed position in which the distal anchor is disposed distal to the distal tip of the needle. The distal portion of the artificial chordae extends through the partial circumferential slit and through the central lumen within both the distal portion and the proximal portion of the tubular body and with the proximal portion proximal to the proximal end of the distal anchor. The distal anchor is reconfigurable from the delivery configuration to a deployed configuration when the distal anchor is in the deployed position shortening drawing distal portion of the artificial through the distal anchor to draw the distal end and the proximal end of the tubular body towards the partial circumferential slit to bend each of the distal portion and the proximal portion of the tubular body into a loop.

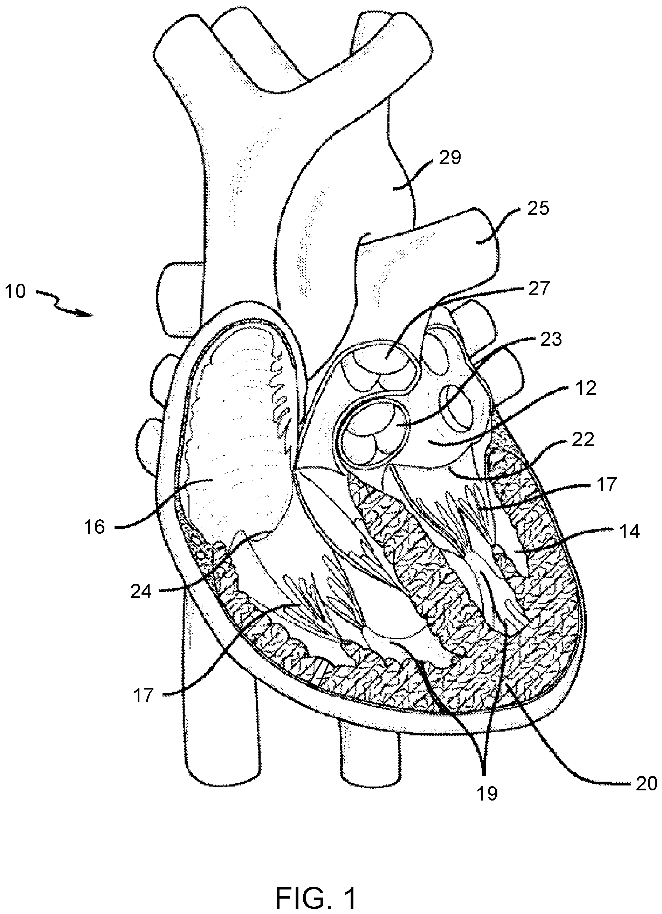

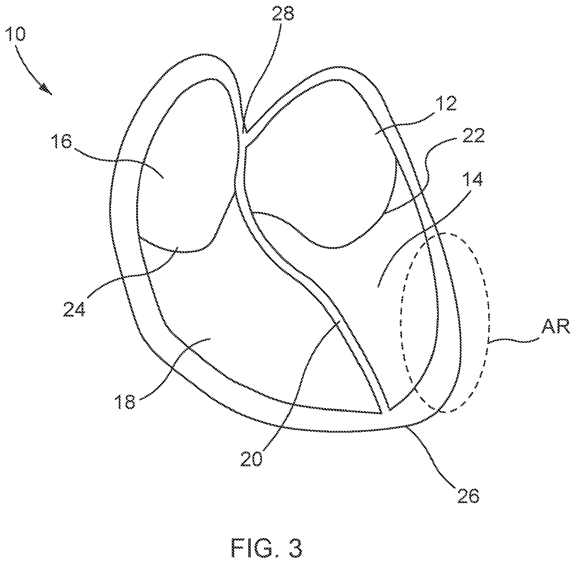

As illustrated in FIG. 1, the human heart 10 has four chambers, which include two upper chambers denoted as atria 12, 16 and two lower chambers denoted as ventricles 14, 18. A septum 20 (see, e.g., FIG. 3) divides the heart 10 and separates the left atrium 12 and left ventricle 14 from the right atrium 16 and right ventricle 18. The heart further contains four valves 22, 23, 26, and 27. The valves function to maintain the pressure and unidirectional flow of blood through the body and to prevent blood from leaking back into a chamber from which it has been pumped.

Two valves separate the atria 12, 16 from the ventricles 14, 18, denoted as atrioventricular valves. The mitral valve 22, also known as the left atrioventricular valve, controls the passage of oxygenated blood from the left atrium 12 to the left ventricle 14. A second valve, the aortic valve 23, separates the left ventricle 14 from the aortic artery (aorta) 29, which delivers oxygenated blood via the circulation to the entire body. The aortic valve 23 and mitral valve 22 are part of the "left" heart, which controls the flow of oxygen-rich blood from the lungs to the body. The right atrioventricular valve, the tricuspid valve 24, controls passage of deoxygenated blood into the right ventricle 18. A fourth valve, the pulmonary valve 27, separates the right ventricle 18 from the pulmonary artery 25. The right ventricle 18 pumps deoxygenated blood through the pulmonary artery 25 to the lungs wherein the blood is oxygenated and then delivered to the left atrium 12 via the pulmonary vein. Accordingly, the tricuspid valve 24 and pulmonic valve 27 are part of the "right" heart, which control the flow of oxygen-depleted blood from the body to the lungs.

Both the left and right ventricles 14, 18 constitute "pumping" chambers. The aortic valve 23 and pulmonic valve 27 lie between a pumping chamber (ventricle) and a major artery and control the flow of blood out of the ventricles and into the circulation. The aortic valve 23 and pulmonic valve 27 have three cusps, or leaflets, that open and close and thereby function to prevent blood from leaking back into the ventricles after being ejected into the lungs or aorta 29 for circulation.

Both the left and right atria 12, 16 are "receiving" chambers. The mitral valve 22 and tricuspid valve 24, therefore, lie between a receiving chamber (atrium) and a ventricle so as to control the flow of blood from the atria to the ventricles and prevent blood from leaking back into the atrium during ejection from the ventricle. Both the mitral valve 22 and tricuspid valve 24 include two or more cusps, or leaflets (not shown in FIG. 1), that are encircled by a variably dense fibrous ring of tissues known as the annulus (not shown in FIG. 1). The valves are anchored to the walls of the ventricles by chordae tendineae (chordae) 17. The chordae tendineae 17 are cord-like tendons that connect the papillary muscles 19 to the leaflets (not shown in FIG. 1) of the mitral valve 22 and tricuspid valve 24 of the heart 10. The papillary muscles 19 are located at the base of the chordae 17 and are within the walls of the ventricles. The papillary muscles 19 do not open or close the valves of the heart, which close passively in response to pressure gradients; rather, the papillary muscles 19 brace the valves against the high pressure needed to circulate the blood throughout the body. Together, the papillary muscles 19 and the chordae tendineae 17 are known as the subvalvular apparatus. The function of the subvalvular apparatus is to keep the valves from prolapsing into the atria when they close.

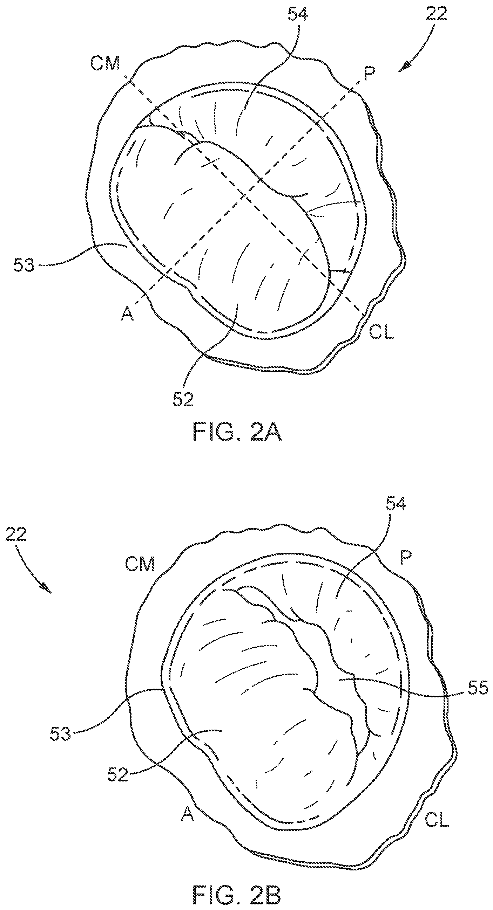

The mitral valve 22 is illustrated in FIG. 2A. The mitral valve 22 includes two leaflets, the anterior leaflet 52 and the posterior leaflet 54, and a diaphanous incomplete ring around the valve, called the annulus 53. The mitral valve 22 has two papillary muscles 19, the anteromedial and the posterolateral papillary muscles (see, e.g., FIG. 1), which attach the leaflets 52, 54 to the walls of the left ventricle 14 via the chordae tendineae 17 (see, e.g., FIG. 1).

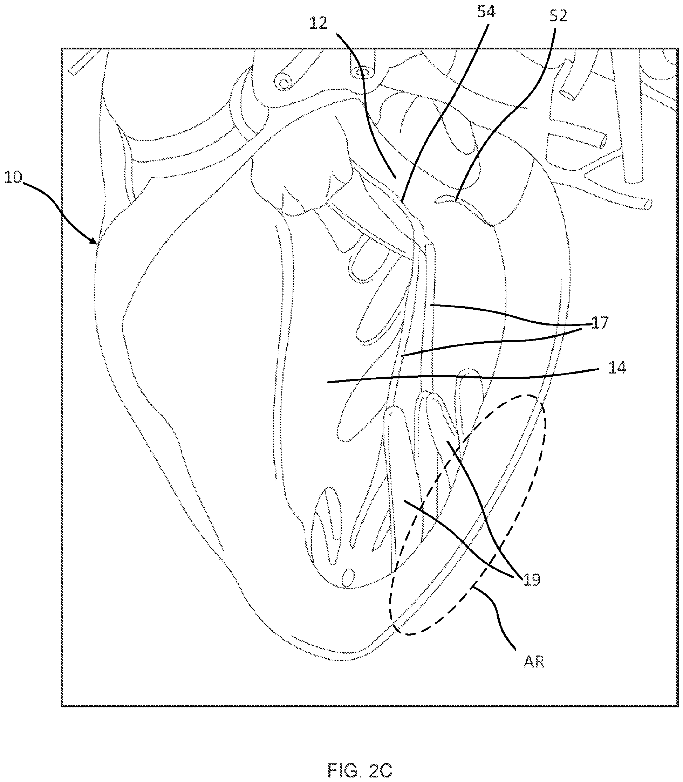

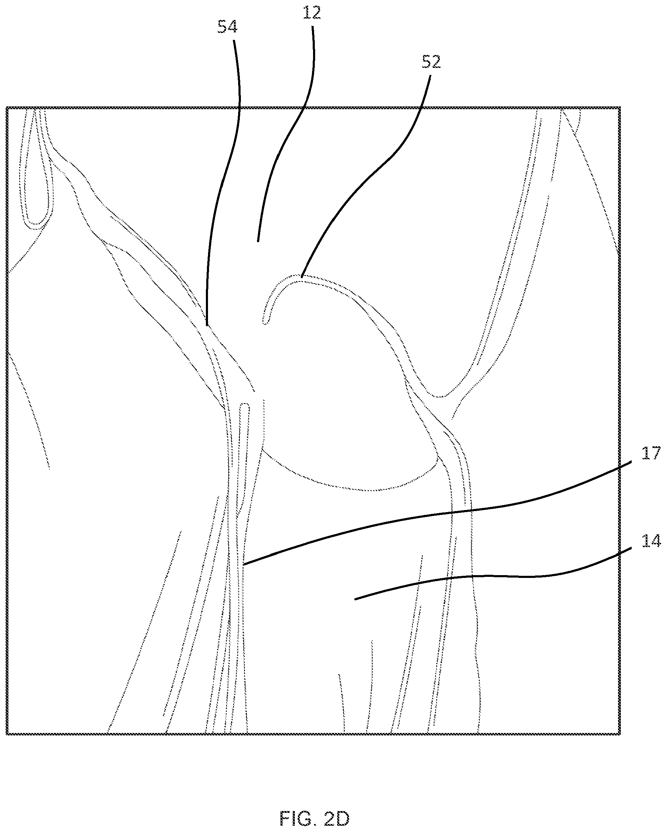

FIG. 2B illustrates a prolapsed mitral valve 22. As can be seen with reference to FIG. 2B-2D, prolapse occurs when a prolapsed segment of a leaflet 52, 54 of the mitral valve 22 is displaced above the plane of the mitral annulus into the left atrium 12 (see FIGS. 2C and 2D) preventing the leaflets from properly sealing together to form the natural plane or line of coaptation between the valve leaflets during systole. Because one or more of the leaflets 52, 54 malfunction, the mitral valve 22 does not close properly, and, therefore, the leaflets 52, 54 fail to coapt. This failure to coapt causes a gap 55 between the leaflets 52, 54 that allows blood to flow back into the left atrium, during systole, while it is being ejected by the left ventricle. As set forth above, there are several different ways a leaflet may malfunction, which can thereby lead to regurgitation.

Mitral valve regurgitation increases the workload on the heart and may lead to very serious conditions if left un-treated, such as decreased ventricular function, pulmonary hypertension, congestive heart failure, permanent heart damage, cardiac arrest, and ultimately death. Since the left heart is primarily responsible for circulating the flow of blood throughout the body, malfunction of the mitral valve 22 is particularly problematic and often life threatening.

As described in detail in PCT International Application No. PCT/US2012/043761 (published as WO 2013/003228 A1) (referred to herein as "the '761 PCT Application), methods and devices are provided for performing non-invasive procedures to repair a cardiac valve, such as a mitral valve. Such procedures include procedures to repair regurgitation that occurs when the leaflets of the mitral valve do not coapt at peak contraction pressures, resulting in an undesired back flow of blood from the ventricle into the atrium. As described in the '761 PCT Application, after the malfunctioning cardiac valve has been assessed and the source of the malfunction verified, a corrective procedure can be performed. Various procedures can be performed in accordance with the methods described therein to effectuate a cardiac valve repair, which will depend on the specific abnormality and the tissues involved.

In one example method not forming part of the present invention, the heart may be accessed through one or more openings made by a small incision(s) in a portion of the body proximal to the thoracic cavity, for example, between one or more of the ribs of the rib cage of a patient, proximate to the xyphoid appendage, or via the abdomen and diaphragm. Access to the thoracic cavity may be sought so as to allow the insertion and use of one or more thorascopic instruments, while access to the abdomen may be sought so as to allow the insertion and use of one or more laparoscopic instruments. Insertion of one or more visualizing instruments may then be followed by transdiaphragmatic access to the heart. Additionally, access to the heart may be gained by direct puncture (i.e., via an appropriately sized needle, for instance an 18 gauge needle) of the heart from the xyphoid region. Accordingly, the one or more incisions should be made in such a manner as to provide an appropriate surgical field and access site to the heart. Access may also be achieved using percutaneous methods. See for instance, Full-Spectrum Cardiac Surgery Through a Minimal Incision Mini-Sternotomy (Lower Half) Technique Doty et al. Annals of Thoracic Surgery 1998; 65(2): 573-7 and Transxiphoid Approach Without Median Stermotomy for the Repair of Atrial Septal Defects, Barbero-Marcial et al. Annals of Thoracic Surgery 1998; 65(3): 771-4.

After prepping and placing the subject under anesthesia, a transesophageal echocardiogram (TEE) (2D or 3D), a transthoracic echocardiogram (TTE), intracardiac echo (ICE), or cardio-optic direct visualization (e.g., via infrared vision from the tip of a 7.5 F catheter) may be performed to assess the heart and its valves.

After a minimally invasive approach is determined to be advisable, one or more incisions are made proximate to the thoracic cavity so as to provide a surgical field of access. The total number and length of the incisions to be made depend on the number and types of the instruments to be used as well as the procedure(s) to be performed. The incision(s) should be made in such a manner so as to be minimally invasive. As referred to herein, the term "minimally invasive" means in a manner by which an interior organ or tissue may be accessed with as little as possible damage being done to the anatomical structure through which entry is sought. Typically, a minimally invasive procedure is one that involves accessing a body cavity by a small incision of, for example, approximately 5 cm or less made in the skin of the body. The incision may be vertical, horizontal, or slightly curved. If the incision is placed along one or more ribs, it should follow the outline of the rib. The opening should extend deep enough to allow access to the thoracic cavity between the ribs or under the sternum and is preferably set close to the rib cage and/or diaphragm, dependent on the entry point chosen.

One or more other incisions may be made proximate to the thoracic cavity to accommodate insertion of a surgical scope so as to allow ready access to and visualization of the heart. The surgical scope may be any type of endoscope, but is typically a thorascope or laparoscope, dependent upon the type of access and scope to be used. At this point, the practitioner can confirm that access of one or more cardiac valves through the apex region of the heart is appropriate for the particular procedure to be performed.

Once a suitable entry point has been established, the surgeon can use one or more sutures to make a series of stiches in one or more concentric circles in the myocardium at the desired location to create a "pursestring" closure. The Seldinger technique can be used to access the left ventricle in the area surrounded by the pursestring suture by puncturing the myocardium with a small sharp hollow needle (a "trocar") with a guidewire in the lumen of the trocar. Once the ventricle has been accessed, the guidewire can be advanced, and the trocar removed. A valved-introducer with dilator extending through the lumen of the valved-introducer can be advanced over the guidewire to gain access to the left ventricle. The guidewire and dilator can be removed and the valved-introducer will maintain hemostasis, with or without a suitable delivery device inserted therein, throughout the procedure. Alternatively the surgeon can make a small incision in the myocardium and insert the valved-introducer into the heart via the incision. Once the valved-introducer is properly placed the pursestring suture is tightened to reduce bleeding around the shaft of the valved-introducer.

A suitable device such as a delivery device described herein, may be advanced into the body and through the valved-introducer in a manner so as to access the left ventricle. The advancement of the device may be performed in conjunction with sonography or direct visualization (e.g., direct transblood visualization). For example, the delivery device may be advanced in conjunction with TEE guidance or ICE so as to facilitate and direct the movement and proper positioning of the device for contacting the appropriate apical region of the heart. Typical procedures for use of echo guidance are set forth in Suematsu, Y., J. Thorac. Cardiovasc. Surg. 2005; 130:1348-1356.

As shown in FIG. 3, one or more chambers, i.e., the left atrium 12, left ventricle 14, right atrium 16, or right ventricle 18 in the heart 10 may be accessed in accordance with the methods disclosed herein. Access into a chamber 12, 14, 16, 18 in the heart 10 may be made at any suitable site of entry but is preferably made in the apex region of the heart, for example, slightly above the apex 26 at the level of the papillary muscles 19 (see also FIG. 2C). Typically, access into the left ventricle 14, for instance, to perform a mitral valve repair, is gained through the process described above performed in the apical region, close to (or slightly skewed toward the left of) the median axis 28 of the heart 10. Typically, access into the right ventricle 18, for instance, to perform a tricuspid valve repair, is gained through the process described above performed in the apical region, close to or slightly skewed toward the right of the median axis 28 of the heart 10. Generally, an apex region of the heart is a bottom region of the heart that is within the left or right ventricular region and is below the mitral valve 22 and tricuspid valve 24 and toward the tip or apex 26 of the heart 10. More specifically, an "apex region" AR of the heart (see FIGS. 2C and 3) is within a few centimeters to the right or to the left of the septum 20 of the heart 10 at or near the level of the papillary muscles 19. Accordingly, the ventricle can be accessed directly via the apex 26, or via an off apex location that is in the apical or apex region AR, but slightly removed from the apex 26, such as via a lateral ventricular wall, a region between the apex 26 and the base of a papillary muscle 19, or even directly at the base of a papillary muscle 19 or above. Typically, the incision made to access the appropriate ventricle of the heart is no longer than about, for example, 0.5 cm. Alternatively, access can be obtained using the Seldinger technique described above.

The mitral valve 22 and tricuspid valve 24 can be divided into three parts - an annulus (see 53 in FIGS. 2A and 2B), leaflets (see 52, 54 in FIGS. 2A and 2B), and a sub-valvular apparatus. The sub-valvular apparatus includes the papillary muscles 19 (see FIG. 1) and the chordae tendineae 17 (see FIG. 1), which can elongate and or rupture. If the valve is functioning properly, when closed, the free margins or edges of the leaflets come together and form a tight junction, the arc of which, in the mitral valve, is known as the line, plane or area of coaptation. Normal mitral and tricuspid valves open when the ventricles relax allowing blood from the atrium to fill the decompressed ventricle. When the ventricle contracts, chordae tendineae properly position the valve leaflets such that the increase in pressure within the ventricle causes the valve to close, thereby preventing blood from leaking into the atrium and assuring that all of the blood leaving the ventricle is ejected through the aortic valve (not shown) and pulmonic valve (not shown) into the arteries of the body. Accordingly, proper function of the valves depends on a complex interplay between the annulus, leaflets, and subvalvular apparatus. Lesions in any of these components can cause the valve to dysfunction and thereby lead to valve regurgitation. As set forth above, regurgitation occurs when the leaflets do not coapt properly at peak contraction pressures. As a result, an undesired back flow of blood from the ventricle into the atrium occurs.

Although the procedures described herein are with reference to repairing a cardiac mitral valve or tricuspid valve by the implantation of one or more artificial chordae, the methods presented are readily adaptable for various types of leaflet and annular repair procedures. In general, the methods herein will be described with reference to a mitral valve 22.

In some examples not forming part of the present invention, a method includes the implantation of one or more artificial chordae tendineae into one or more leaflets (e.g., 52, 54 in FIGS. 2A and 2B) of a malfunctioning mitral valve 22 and/or tricuspid valve 24. After an appropriate incision has been made in the apex region of the heart, for example, in the apex 26, a delivery device can be introduced into, for example, the left ventricle 14 of the heart and advanced in such a manner so as to contact one or more cardiac tissues (for instance, a leaflet, an annulus, a cord, a papillary muscle, or the like) that are in need of repair. Sonic guidance, for instance, TEE guidance or ICE, may be used to assist in the advancement of the device into the ventricle, the proper positioning of the distal tip of the device on the proximal side of the leaflet and, if necessary, the grasping of the cardiac tissue with the device. Direct transblood visualization may also be used.

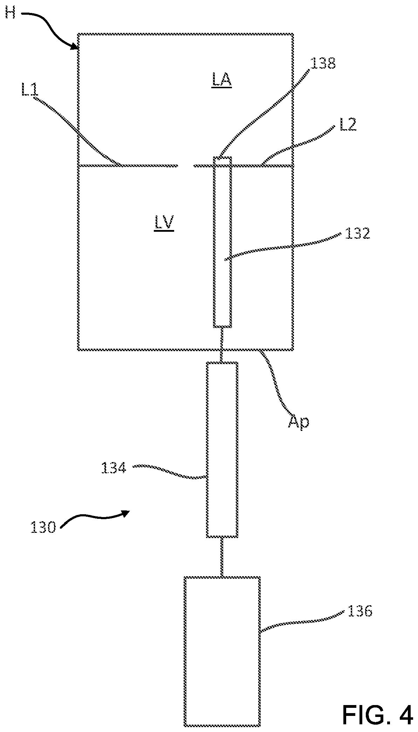

FIG. 4 is a schematic illustration of a portion of a heart with a delivery device inserted therein, according to an example useful for understanding the present invention. The delivery device 130 can include a distal end portion 132 configured to be inserted into a heart H, an elongate portion 134 coupled to the distal end portion 132, and a proximal end portion 136. The distal end portion 132 of the delivery device 130 can include a piercing member (not shown) and an anchor support portion (not shown). The distal end portion 132 can include other features to enable the delivery device 130 to perform various functions, such as, for example, grasping, suctioning, irrigating, cutting, suturing, or otherwise engaging a cardiac tissue.

The proximal end portion 136 can include, for example, a handle that can be used by the user/operator to manipulate movement of the delivery device 130 and/or to actuate the delivery device 130. The proximal end portion 136 can also include control features and/or components that can be used to actuate various functions of the delivery device 130. The proximal end portion 136 can also include a holding device or member that can be used to hold and control a tether (e.g., suture, cord or wire) extending from a distal anchor (described in more detail below) during deployment of the distal anchor.

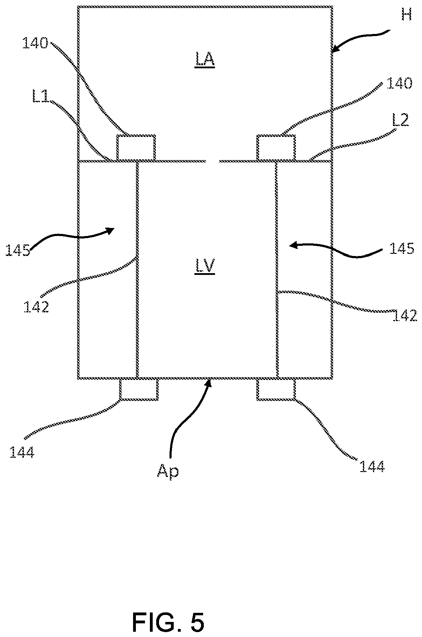

Using, for example, ultrasound guidance (real-time transesophageal echocardiography), the delivery device 130 can be inserted through an access port at the apex Ap (or near the apex) of the heart H and guided through the left ventricle LV and into contact with a proximal side of a mitral valve leaflet L1 (or L2), shown in FIGS. 4 and 5, at a location where the user/operator has determined that a repair is needed. Typically, this would be a prolapsed segment of the body of the anterior or posterior leaflet, i.e. in a location where the valve has prolapsed as a result of a broken or elongated chord. The distal end portion 130 of the delivery device 130 can be used to puncture or form an opening in the valve leaflet L1 and/or the valve leaflet L2. For example, as shown in FIG. 4, the piercing member at a distal tip 138 can be used to puncture or pierce through the leaflet L2. This can be done with or without grasping, capturing, or otherwise immobilizing the prolapsed segment of the leaflet.

The distal tip 138 of the delivery device 130 can be inserted through the puncture site or opening and positioned on a distal side of the leaflet L2 and within the left atrium LA. When the distal tip 138 is in the desired positon, the delivery device 130 can be actuated to insert a distal anchor 140 or form a distal anchor 140 (see, FIG. 5) on the distal side of the leaflet L2 within the left atrium LA of the heart H. In some examples, the distal anchor 140 can include a suture or a suture/guide wire combination that can form into a knot upon actuation of the delivery device 130. For example, the distal anchor 140 includes a large or bulky knot made of ePTFE suture or other appropriate material that is formed by the delivery device 130 and that attains a significant size in the left atrium LA, above the leaflet L2. The knot can be in the form of one or more multi-turn coils of the suture or other material used to form the tether (described in more detail below), which coils can be changed from an elongated configuration to a knot configuration by approximating opposite ends of the coil(s) towards each other, to form one or more loops. In some examples, the distal anchor 140 includes an anchor member that is deployed into the left atrium LA above the leaflet L2 upon actuation of the delivery device 130.

The distal anchor 140, whether formed by the delivery device 130 or deployed by the delivery device 130 can be coupled to a tether 142 extending proximally from the distal anchor 140 and secured to the proximal end portion 136 of the delivery device 130. Alternatively, the distal anchor 140 and the tether 142 can be all one component (i.e., ePTFE suture) where the distal anchor 140 is formed by altering the shape of the tether 142 from a first position to a second position. As described above, the proximal end portion 136 of the delivery device 130 can include a holding device (not shown) that can be used to secure and control the tether 142 during delivery and deployment of the distal anchor 140.

As shown in FIG. 5, after the distal anchor 140 has been deployed or formed, the delivery device 130 can be withdrawn from the heart H. The length of the tether 142 between the distal anchor 140 and the opening in the heart can be adjusted, as discussed above, until the desired length is established (i.e. prolapse of the leaflet is prevented, but the leaflet can still move distally sufficient to coapt with the other leaflet(s)). The proximal end of the tether 142 can then be secured to an outer surface of the heart H at, for example, the apex Ap region, with a proximal anchor 144. The proximal anchor 144 can be, for example, a pledget, one or more knots, or other suitable anchoring device.

The above procedure can be performed multiple times on the same leaflet, and/or can be performed on the other mitral valve leaflet L1 in the same manner. The result can thus be that two or more anchor-tether apparatuses 145 are each anchored on a distal side of a leaflet L1, L2 with a distal anchor 140 and secured to the apex Ap region of the heart H with a proximal anchor 144 via the tether 142. Thus, each anchor-tether apparatus 145 can secure the top of the leaflet L 1, L2 to the apex Ap region of the heart H, functioning as an artificial chordae or neochord.

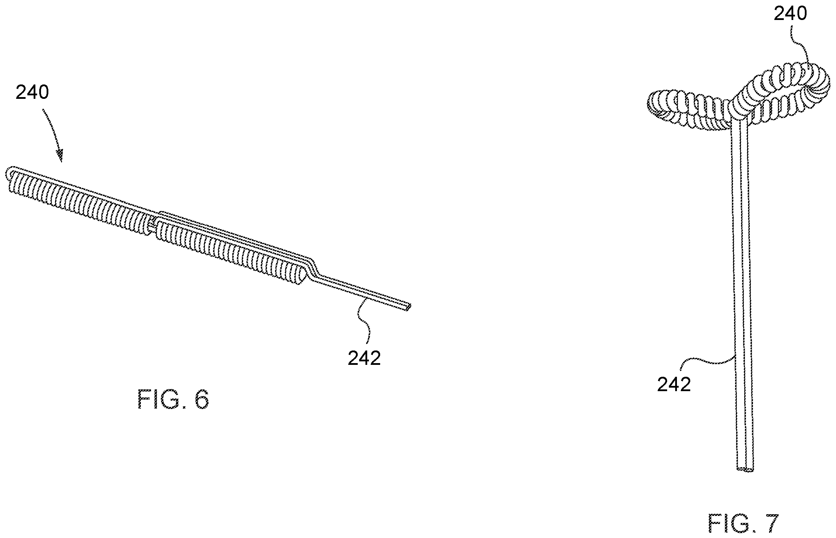

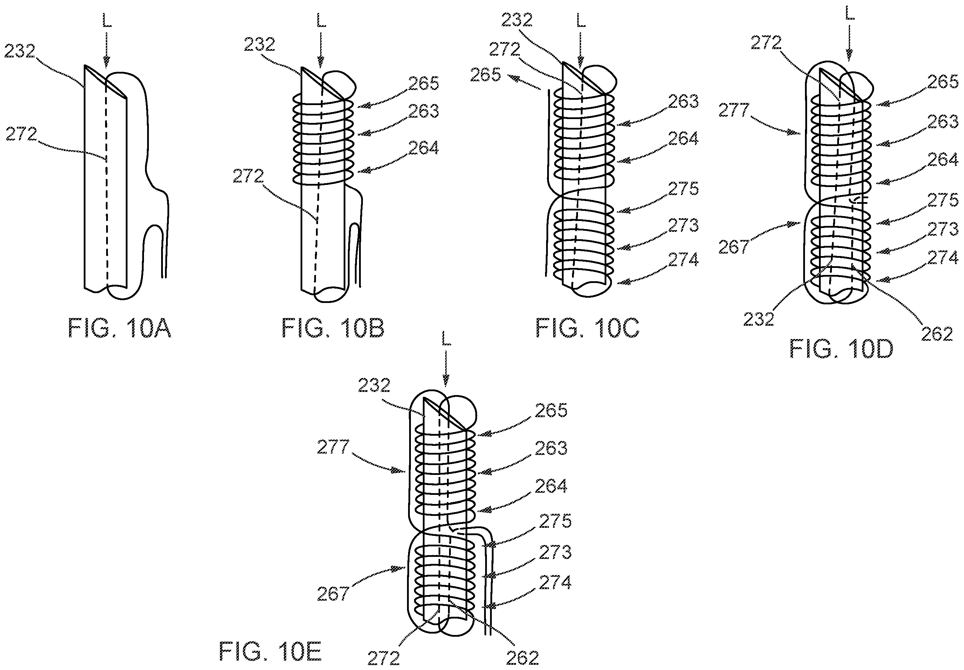

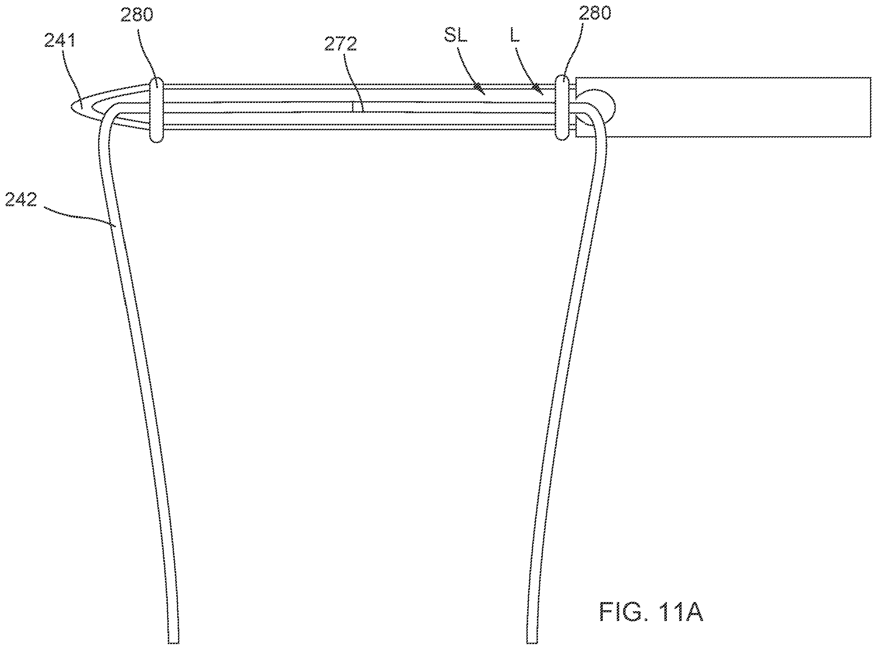

The distal anchor(s) described above, can be a variety of different shapes, sizes and configurations that can be delivered and deployed within a heart using a variety of different delivery devices. In some examples, a distal anchor can include a knot that can be formed/deployed using a distal anchor delivery device such as, for example, a delivery device as shown and described in U.S. Provisional Patent Application Serial No. 62/236,225 filed October 2, 2015 ("the '225 application"). For example, the distal anchor is a large or bulky knot. As shown in FIGS. 6 and 7, a distal anchor 240 includes a suture 242 that is formed into an elongated coiled configuration as shown in FIG. 6, with two strands of the suture 242 extending from the elongated coiled configuration of the distal anchor 240. For example, the distal anchor can be in the form of one or more multi-turn coils of the suture 242. The formation of the distal anchor 240 in the elongated coiled configuration is described in more detail below with respect to FIGS. 10A-10E and 11A-11H. The distal anchor 240 can be deployed into a heart and transformed, reconfigured, moved or otherwise changed to a coiled knot configuration as shown in FIG. 7. For example, as discussed above for distal anchor 140, the distal anchor 240 (e.g., knot) can be changed from the elongated coiled configuration to the knot configuration by approximating opposite ends of the coil(s) towards each other, to form one or more loops. For example, the two strands or lengths of the suture 242 extending from a medial portion of the distal anchor 240 (e.g., the elongated coiled portion of the suture 242) can extend through the delivery device and when the two proximal ends of the suture 242 are pulled proximally, the opposite ends of the coiled portions are pulled towards each other to form the loops.

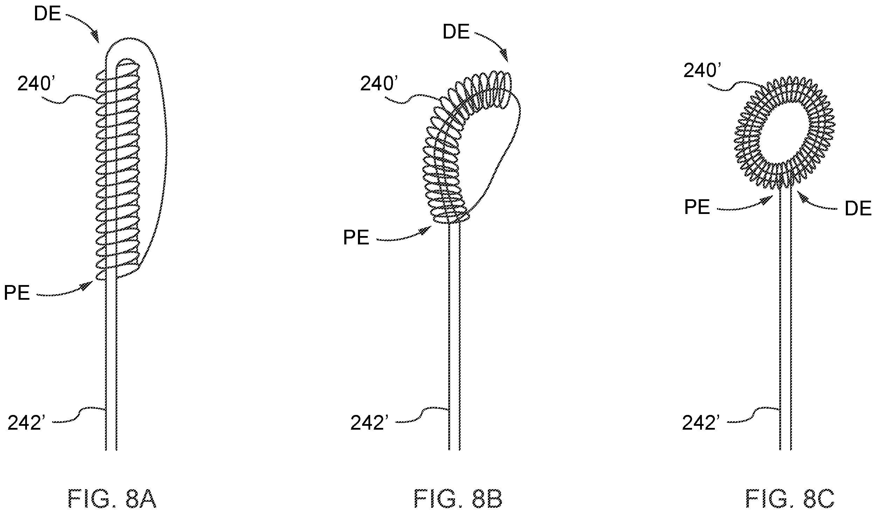

FIGS. 8A-8C illustrate the sequence described above with respect to the distal anchor 240 transitioning from an elongated configuration to a knot configuration, however for ease of illustration, only a single coil and loop are shown and described. As shown in FIG. 8A, the distal anchor 240 is in a coiled, elongated formation (e.g., a preformed knot) configured for delivery to a heart. To form the knot configuration (as shown in FIG. 8C), a proximal end of the suture 242 is pulled proximally to deflect the distal end DE of the coil laterally with respect to the proximal end PE of the coil and to draw the proximal end PE of the coil and the distal end DE of the coil towards each other to form a loop L, as illustrated in FIGS. 8B and 8C.

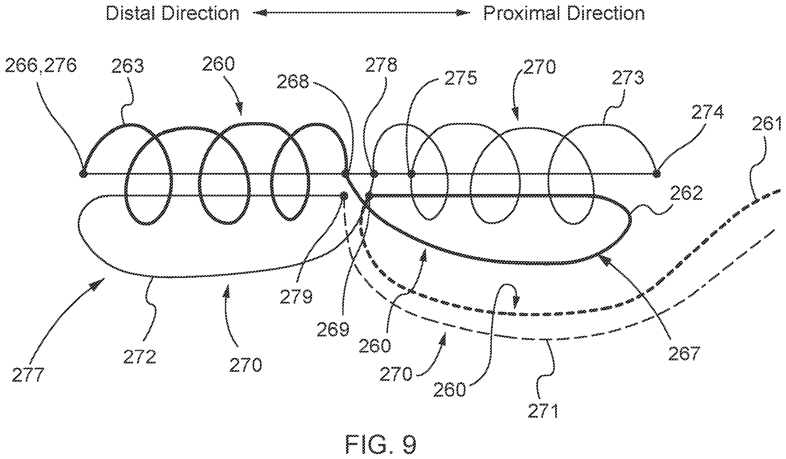

FIG. 9 shows a schematic illustration of a distal anchor 240 of FIG. 6 in the elongated coiled configuration. For ease of explanation, the distal anchor 240 is shown and described with reference to a first section 260 of the suture 242 and a second section 270 of the suture 242. The first section 260 has a first portion 261 (as shown in dashed line for ease of illustration) and a second portion 262 including a first coil 263 formed of multiple turns about the exterior of the distal end portion 232 of the delivery device 230. The first coil 263 has a proximal end 264 and a distal end 265. The second portion 262 of the first section 260 has a first end 266 at the distal end 265 of the first coil 263.

The second section 270 has a second portion 272 with a first end 276, and extends proximally from the first end 266 of the first section 260 through an interior of the first coil 263 (and through the lumen of the distal end portion 232 of the deliver device 230) to the proximal end 264 of the first coil 263. The second section 270 also includes a loop forming segment 277 that extends distally from a first end 278 of the loop forming segment 277 at the proximal end 264 of the first coil 263 along the outside of the first coil 263 to the distal end 265 of the first coil 263, and extends proximally through the interior of the first coil 263 (and through the lumen of the distal end portion 232 of the deliver device 230) to the proximal end 264 of the first coil 263 at a second end 279 of the loop forming segment 277.

The second portion 272 of the second section 270 includes a second coil 273 formed of multiple turns about the exterior of the distal end portion 232 of the delivery device 230 proximal to the first coil 263, and has a proximal end 274 and a distal end 275. The second portion 272 of the second section 270 extends proximally from the first end 276 of the second portion 272 through the interior of the second coil 273 (and as shown in FIGS. 10A-10E, e.g., through the lumen of the distal end portion 232 of the deliver device 230) to the proximal end 274 of the second coil 273. The first end 278 of the loop forming segment 277 of the second portion 272 of the second section 270 extends from the distal end 275 of the second coil 273.

The second portion 262 of the first section 260 has a loop forming segment 267 that extends from a first end 268 of the loop forming segment 267 of the second portion 262 of the first section 260 proximally from the proximal end 264 of the first coil 263 along the outside of the second coil 273 to the proximal end 274 of the second coil 273 and extends distally through the interior of the second coil 273 (and as shown in FIGS. 10A-10E, e.g., through the lumen of the distal end portion 232 of the deliver device 230) to the distal end 275 of the second coil 273 at a second end 269 of the loop forming segment 267 of the second portion 262 of the first section 260. The first portion 261 of the first section 260 extends proximally from the second end 269 of the loop forming segment 267 of the second portion 262 of the first section 260.

FIGS. 10A-10E illustrate in sequence the formation of the distal anchor 240 of FIGS. 6 and 9 about an exterior of a needle of a delivery device (not shown) and in an elongated coiled configuration (FIG. 10E). The needle defines a lumen L therethrough and a slot (not shown) in communication with the lumen L. To form the distal anchor 240 about the needle, the second portion 272 of the second section 270 of the suture 242 is routed through the lumen L of the needle (see e.g., FIG. 10A). Next, the second portion 262 of the first section 260 of the suture 242 is wrapped about the needle to form the first coil 263 (see e.g., FIG. 10B). Similarly, the second portion 272 of the second section 270 of the suture 242 is wrapped about the needle proximate to the first coil 263 to form the second coil 273 (see e.g., FIG. 10C).

After formation of the first coil 263 and the second coil 273 about the needle, the loop forming segment 267 of the second portion 262 of the first section 260 is formed by routing proximally the section portion 262 of the first section 260 of the suture 242 from the proximal end 264 of and exterior to the first coil 263 towards the proximal end 274 of the second coil 273 (see e.g., FIG. 10C), and then extending distally through the interior of the second coil (see e.g., FIG. 10D). In a similar manner, the loop forming segment 277 of the second portion 272 of the second section 270 is formed by routing distally the second portion 272 of the second section 270 of the suture 242 from the distal end 275 of the second coil 273 towards the distal end 265 of the first coil 263 (see e.g., FIG. 10C), and then extending proximally through the interior of the first coil 263 to the proximal end 264 of the first coil 263 (see e.g., FIG. 10D). The first portion 261 of the first section 260 of the suture 242 and the first portion 271 (as shown in dashed line for ease of illustration) of the second section 270 of the suture 242 extends from the lumen L of the needle through the slot (not shown) of the needle to an area external to the needle such that each portion 261, 271 can be manipulated (e.g., pulled proximally) to form the knot, as described above.

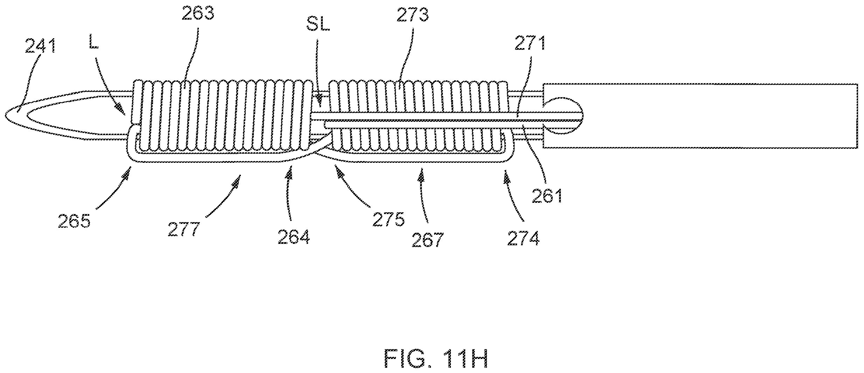

FIGS. 11A-11H illustrate an example method of forming the distal anchor 240 of FIGS. 6 and 7 in the elongated coiled configuration (FIG. 11H) about an exterior of a needle 241. The needle 241 defines an interior lumen L, and a distal portion of the needle 241 includes a slot SL in communication with the lumen. As shown in FIG. 11A, the second portion 272 of the second section 270 of the suture 242 is routed through the slot SL of the needle 241 and between the knot rings 280. The knot rings 280 (e.g., silicon O-rings) are disposed about the suture 242 and the needle 241 to secure the suture 242 to and within the slot SL of the needle 241. In this manner, the knot rings 280 define the outer edges (or the distal end 265 of the first coil 263 and the proximal end 274 of the second coil 273) of the distal anchor 240, and can secure the suture 242 such that the first coil 263 and the second coil 273 can be formed about the needle 241.

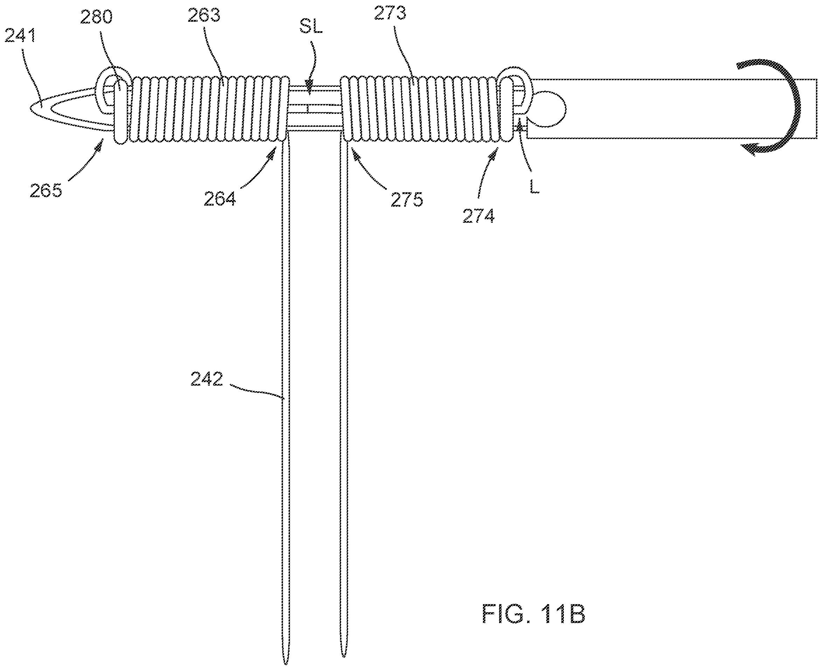

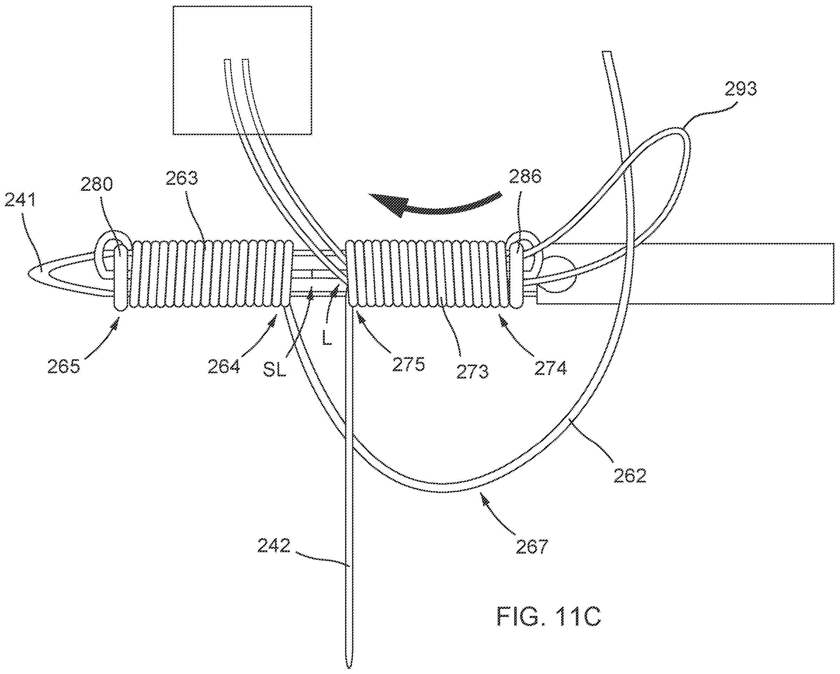

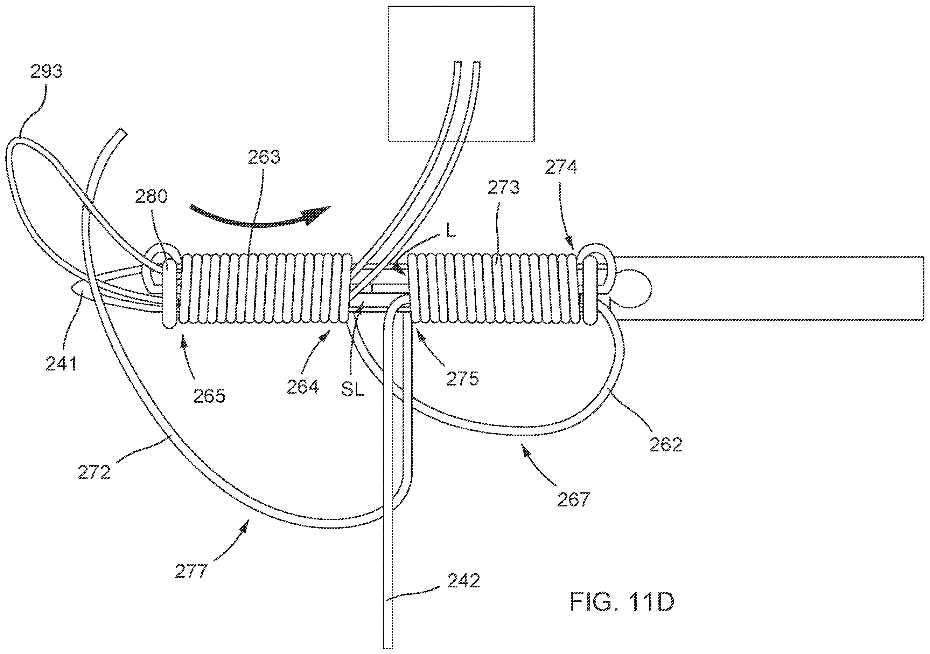

To form the first coil 263 and the second coil 273, the needle 241 is rotated such that the free ends (or the second portion 262 of the first section 260 and the second portion 272 of the second section 270) of the suture 242 form multiple turns about the exterior of the needle 241, as shown in FIG. 11B. Next, the loop forming segment 267 of the second portion 262 of the first section 260 is formed by routing proximally the second portion 262 of the first section 260 of the suture 242 from the proximal end 264 of and exterior to the first coil 263 towards the proximal end 274 of the second coil 273, and then extending distally through the interior of the second coil 273 to the distal end 275 of the second coil 273, as shown in FIG. 11C. In a similar manner, the loop forming segment 277 of the second portion 272 of the second section 270 is formed by routing distally the second portion 272 of the second section 270 of the suture 242 from the distal end 275 of the second coil 273 towards the distal end 265 of the first coil 263, and then extending proximally through the interior of the first coil 263 to the proximal end 264 of the first coil 263, as shown in FIG. 11D.

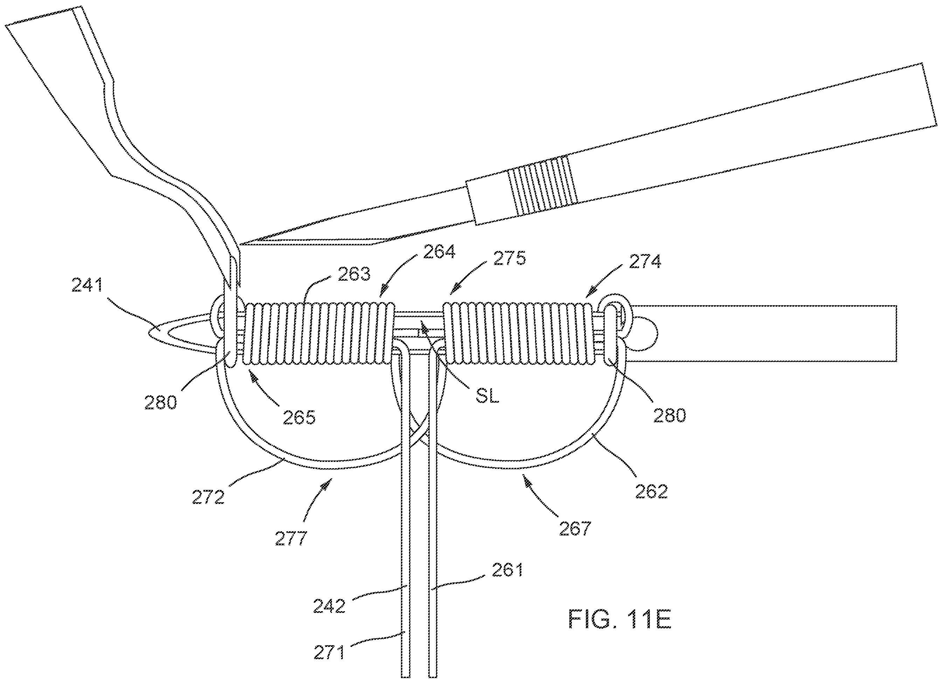

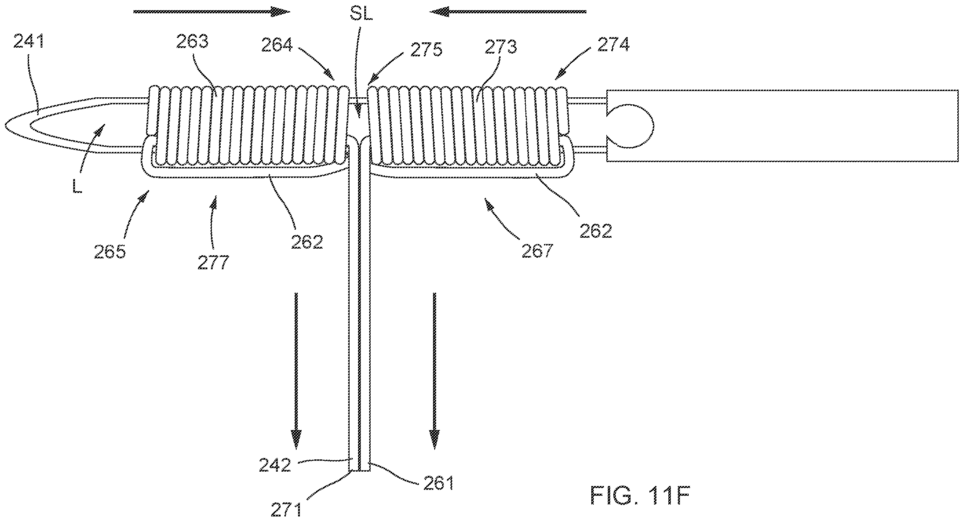

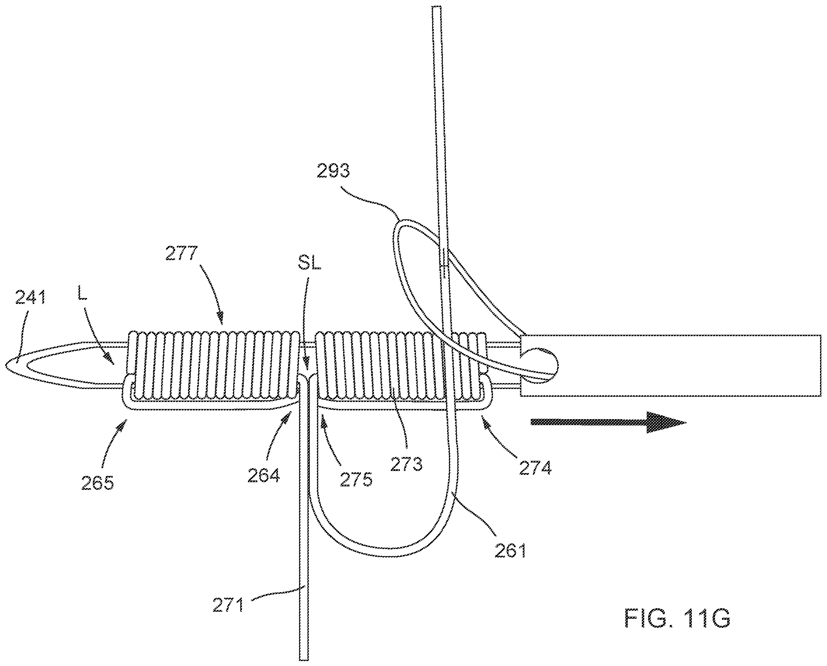

To further prepare the distal anchor 240 for delivery to a heart, as described in previous examples, the loop forming segments can be shortened and/or tightened by pulling the first portion 261 of the first section 260 of the suture 242 and the first portion 271 of the second section 270 of the suture 242. Such a configuration is shown in FIG. 11E. Once the loop forming segments 267, 277 are formed, the knot rings 280 can be removed from the needle 241 and the suture 242. Upon removal of the knot rings 280, the loop forming segments 267, 277 can be further shortened or tightened, as shown in FIG. 11F and 11G. Next, the first portion 261 of the first section 260 of the suture 242 and the first portion 271 of the second section 270 of the suture 242 can be routed proximally into a distal end of the interior lumen L of the needle 241 and proximally through the interior lumen L, as shown in FIGS. 11G and 11H.

In some examples, a snare 293 can be used to facilitate routing of the suture 242 and forming of the distal anchor 242, as illustrated in FIGS. 11C, 11D, and 11G. For example, the snare 223 can be used to route the first portion 261 of the first section 260 and the first portion 271 of the second section 270 into the interior lumen L of the needle and proximally through the interior lumen L.

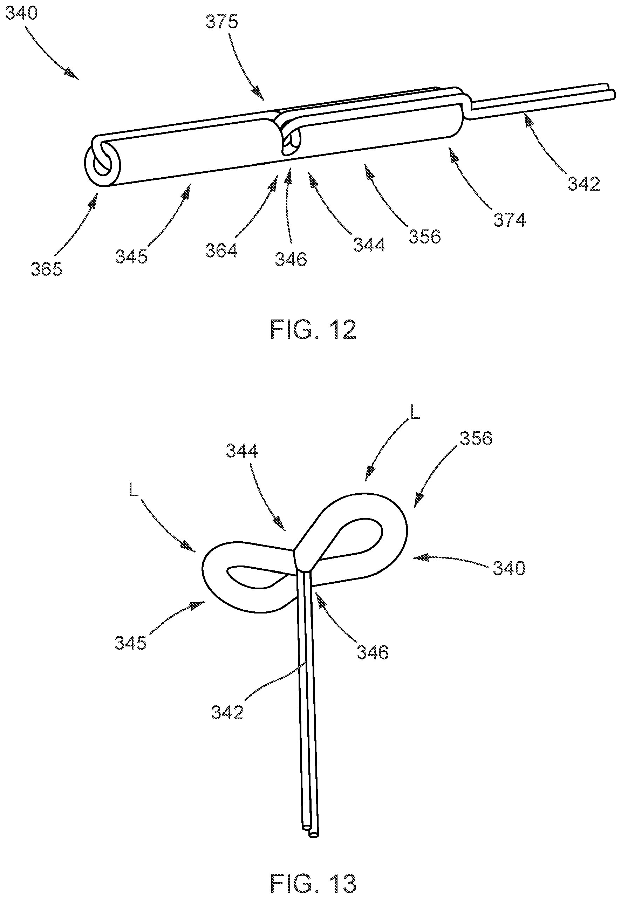

In another example of a distal anchor not forming part of the present invention, the circumferential windings of the knot in the knot distal anchor 240 described above are replaced by a single flexible tube. Such an example of a distal anchor is illustrated in FIGS. 12 and 13. FIG. 12 illustrates a distal anchor 340 in an elongated delivery configuration, and FIG. 13 illustrates the distal anchor 340 in a deployed configuration. In this example, the flexible tube 344 has a distal portion 345, and a proximal portion 356, and a slit 346 separating the distal portion 345 from the proximal portion 356. In an alternative example, instead of a single flexible tube 344, the anchor 340 can be formed with a separate distal tube and proximal tube (not shown), separated by a gap, rather than a partial circumference slit in a middle portion of a single flexible tube, as shown in FIG 12. In this example, as shown, the suture 342 is routed into and through the slit 346, into a lumen of the flexible tube 344, extending distally through the lumen from the slit 346 through and out of a distal end 365 of the distal portion 345, then extending proximally along the exterior of the flexible tube 344 towards a proximal end of the distal portion 345, through the slit 346 and through the lumen of the proximal portion 356 from the distal end 344 of the proximal portion 356 to the proximal end 374 of the proximal portion 356 and out the proximal end 374, then extending distally along the exterior of the proximal portion 356 of the flexible tube 344 towards the slit 346, and optionally looped around a portion of the suture 342 located within or adjacent to the slit 346, as shown in FIG. 12.

Similar to the knot distal anchor 240 described above, the distal anchor 340 can be deployed in a similar manner using any of the delivery devices described in the '225 application. For example, the distal anchor 340 can be delivered in the elongate configuration (FIG. 12) and moved to the deployed configuration (FIG. 13) by pulling the suture strands 342 proximally to deflect the distal end 365 of the distal portion 345 of the flexible tube 344 laterally with respect to a proximal end 364 of the distal portion 345 of the flexible tube 344 to draw the proximal end 364 and the distal end 365 of the distal portion 345 of the flexible tube 344 towards each other to form a loop L as shown in FIG. 13. Similarly, the suture strands 342 can be pulled proximally to deflect the distal end 375 of the proximal portion 356 of the flexible tube 344 laterally with respect to a proximal end 374 of the proximal portion 356 of the flexible tube 344 to draw the proximal end 374 and the distal end 375 of the proximal portion 356 of the flexible tube 344 towards each other to form a loop L as shown in FIG. 13.

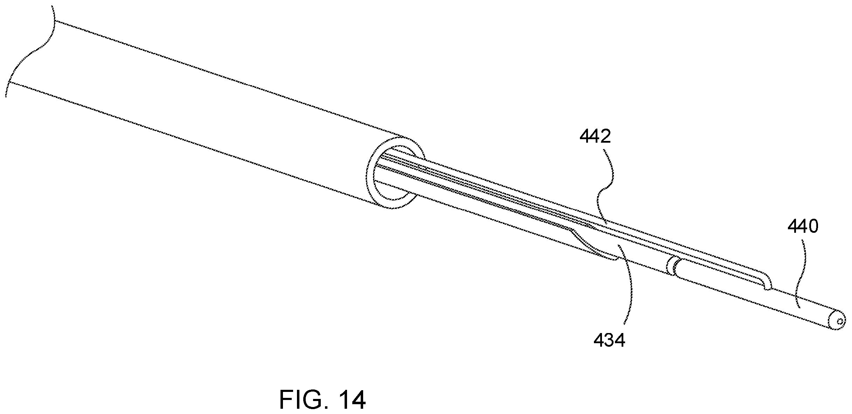

In another example of a distal anchor not forming part of the present invention, the circumferential windings of the knot in the knot distal anchor 240 described above are replaced by a T-fastener, as shown in an elongated delivery configuration in FIG. 14. Similar to the knot distal anchor 240 described above, the distal anchor (or T-fastener) 440 can be deployed in a similar manner using any of the delivery devices described in the '225 application. For example, the distal anchor 440 can be coupled to a suture or sutures 442 and removably coupled to or otherwise in operable contact with a pusher 434. The distal anchor 440 can be delivered in the elongate configuration and moved to the deployed configuration by pulling the suture 442 proximally to rotate the distal anchor 440 such that the distal anchor 440 is non-parallel with respect to the pusher 434, the distal end portion of the delivery device 430, and/or the suture 442. Simultaneously, the distal anchor 440 can be decoupled or otherwise separated from (not shown) the pusher 434 as the pusher 434 is moved distally relative to a handle (not shown) of the delivery device and the suture 442 is pulled proximally.

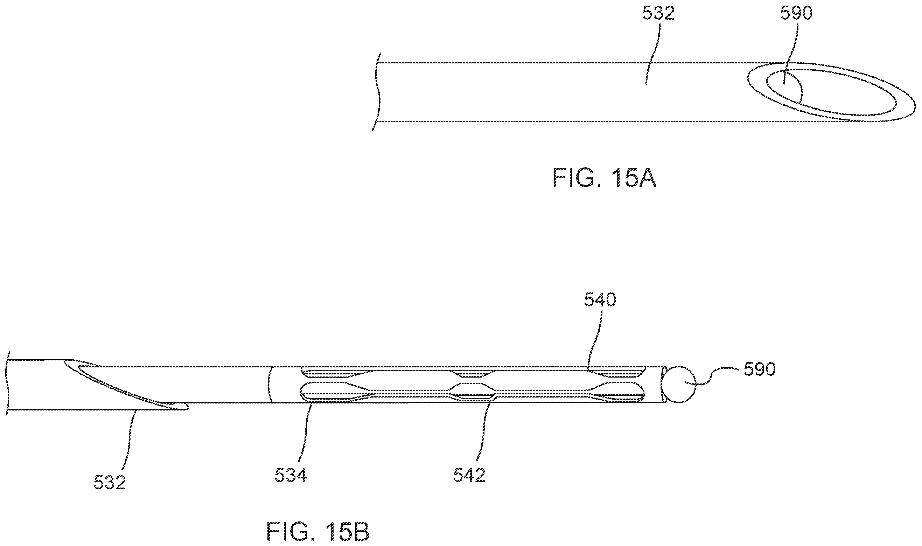

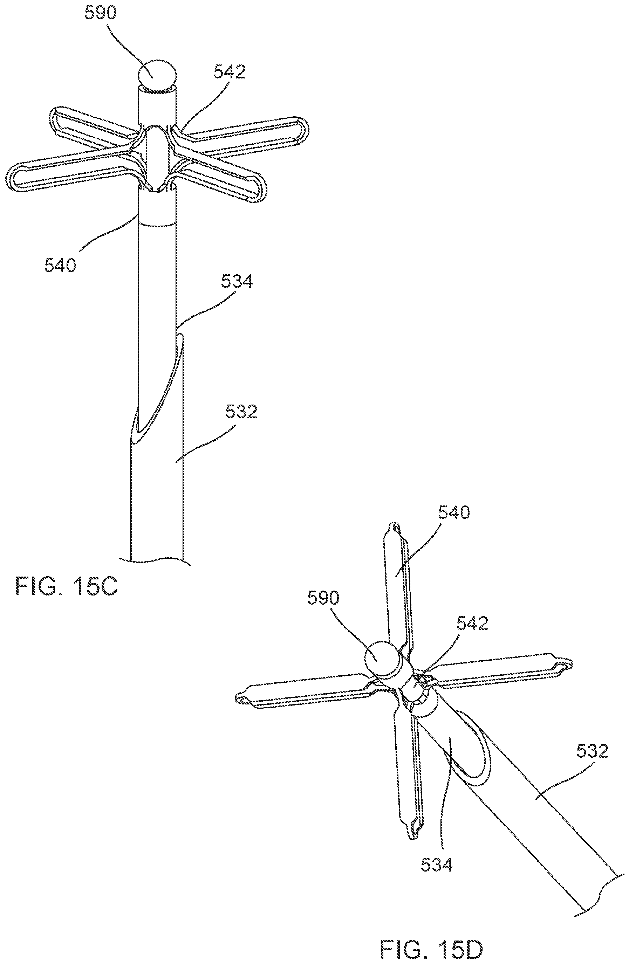

In another example of a distal anchor not forming part of the present invention, the circumferential windings of the knot in the knot distal anchor 240 described above are replaced by an expandable distal anchor, as shown in FIGS. 15A-15D. FIG. 15A illustrates the distal anchor 540 in an elongated delivery configuration disposed within a lumen defined by and disposed through the distal end portion 532 of the delivery device. FIG. 15B illustrates the distal anchor 540 in the elongated deliver configuration and disposed outside of and distal to the distal end portion 532 of the delivery device. FIGS. 15C and 15D illustrate the distal anchor 540 in a deployed configuration in side and perspective view, respectively. Similar to the knot distal anchor 240 described above, the distal anchor 540 can be deployed in a similar manner using any of the delivery devices described in the '225 application. For example, the distal anchor 540 can be coupled to a suture 542 (or disposed about the suture 542 such that the suture 542 extends through a lumen defined by the distal anchor 540) having a stopper 590 disposed at a distal end of the suture 542. The suture 542 is removably coupled to or otherwise in operable contact with a pusher 534. The distal anchor 540 can be delivered in the elongate configuration (see e.g., FIGS. 15A and 15B) and moved to the deployed configuration by pulling the suture 542 proximally and/or moving the pusher 534 distally as shown in FIGS. 15C and 15D. In this manner, both the stopper 590 and the pusher 534 can collectively facilitate the transition of the distal anchor 540 from the elongated delivery configuration to the radially expanded deployed configuration.

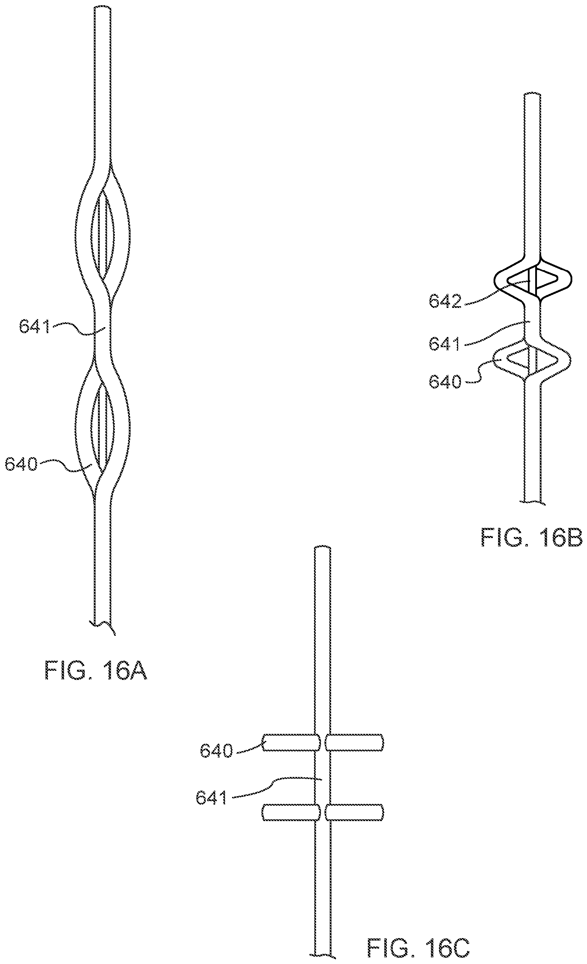

In another example of a distal anchor not forming part of the present invention, the expandable distal anchor 540 described above is replaced by a double expandable distal anchor, as shown in FIGS. 16A-16C. FIG. 16A illustrates the distal anchor 640 in an elongated delivery configuration. FIG. 16B illustrates the distal anchor 640 in a partially deployed configuration. FIG. 16C illustrates the distal anchor 640 in a deployed configuration. Similar to expandable distal anchor 540 described above, the distal anchor 640 can be deployed in a similar manner using, for example, any of the delivery devices described in the '225 application. For example, the distal anchor 640 can be disposed about a suture 642 such that the suture 642 extends through a lumen defined by the distal anchor 640, and removably coupled to or otherwise in operable contact with a pusher (not shown). The distal anchor 640 can be delivered in the elongate configuration (see e.g., FIG. 16A) and moved to the deployed configuration (see e.g., FIGS. 16B and 16C) by pulling the suture 642 proximally and/or moving the pusher (not shown) distally. In this example, the distal anchor 640 includes two slits. As the suture 642 is pulled proximally and/or the pusher (not shown) is moved distally, the slits facilitate expansion of two portions of the distal anchor 640, as shown in FIG. 16B. In its deployed configuration, the ends of the first slit and the ends of the second slit are brought into or nearly into contact with one another, as shown in FIG. 16C to maximize the expansion of the two portions of the distal anchor 640.

In use, in some examples, the distal anchor 640 is delivered in the elongate configuration (see e.g., FIG. 16A) through an opening in a leaflet (e.g., a prolapsed segment of a native mitral valve leaflet) until a medial portion 641 of the distal anchor 640 is disposed in the opening of the leaflet and a first slit is disposed in the left atrium of the heart and the second slit is disposed in the left ventricle of the heart. The distal anchor 640 is then moved into its deployed configuration (see e.g., FIGS. 16B and 16C) such that the two portions (defined in part by the slits) expand radially and/or laterally. In this manner, the two portions of the distal anchor 640 can collectively grab, grasp, sandwich, or otherwise maintain a portion of the native valve leaflet therebetween. In addition to operably coupling the distal anchor 640 to the native valve leaflet, the distal anchor 640 when deployed provides a seal across the opening of the leaflet to prevent or otherwise limit any fluid flow through the opening. In some examples, the portions of the distal anchor 640 can be deployed (e.g., expanded) simultaneously, while in other examples the portions of the distal anchor 640 can be deployed sequentially, e.g., the distal portion can be deployed at a first time and the proximal portion can be deployed at a second time after the first time, or vice versa.

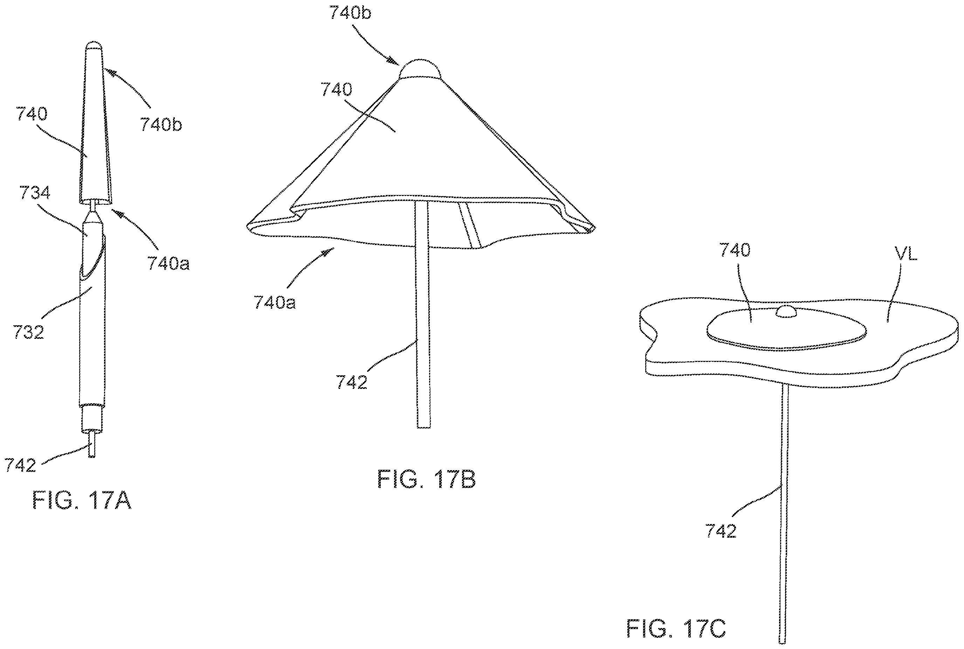

In another example of a distal anchor not forming part of the present invention, the circumferential windings of the knot in the knot distal anchor 240 described above are replaced by an expandable distal anchor (or umbrella anchor), as shown in FIGS. 17A-17C. FIG. 17A illustrates the distal anchor 740 in an elongated collapsed delivery configuration and proximate to a distal end portion 732 of a delivery device. FIG. 17B illustrates the distal anchor 740 in a partially deployed configuration. FIG. 17C illustrates the distal anchor 740 in a deployed or expanded configuration and disposed distal to a valve leaflet VL. In this example, during delivery of the distal anchor 740, the interior walls of the distal end portion 732 can retain the distal anchor 740 in its elongated delivery configuration when the distal anchor 740 is disposed within a lumen defined by the distal end portion 732. When in the elongated delivery configuration, an open end portion 740a of the distal anchor 740 is disposed proximal to a rounded distal end 740b of the distal anchor as shown in FIG. 17A. Similar to distal anchor 240 described above, the distal anchor 740 can be deployed in a similar manner using any of the delivery devices described in the '225 application. For example, the distal anchor 740 can be coupled to a suture 742 and removably coupled to or otherwise in operable contact with a pusher 734. The distal anchor 740 can be delivered in the elongate configuration (see e.g., FIG. 17A), and moved to the deployed configuration (see e.g., FIGS. 17B and 17C) by pulling the suture 742 proximally and/or moving the pusher 734 distally. As the distal anchor 740 is moved distally and the open end portion 740a exits the distal end portion 732 of the delivery device, the distal anchor 740 is allowed to expand (i.e., the open end 740a opens) towards its deployed or expanded configuration, as shown in FIG. 17B.

In an alternative example not forming part of the present invention, a distal anchor can be configured similar to the distal anchor 740 except that the distal anchor can be disposed on the suture 742 such that the open end of the umbrella shaped portion is distal to the rounded distal end of the distal anchor. In such an example, the rounded distal end can define a hole through which the suture can be extended and secured. The distal anchor can be formed with for example a shape-memory material such that the distal anchor has a biased expanded or deployed configuration and an elongated collapsed configuration when constrained within a delivery device. The distal anchor can be pushed or moved out of a delivery device with, for example, a pusher device. As the distal anchor exits a distal end of the delivery device, the distal anchor can transition from its elongated collapsed configuration to its expanded, deployed or biased configuration. Said another way, as the distal anchor exits the distal end of the delivery device, the open end of the distal anchor opens to its expanded or biased configuration. In this manner, the distal anchor can transition from its delivery configuration to its deployed configuration as it exits the delivery device.

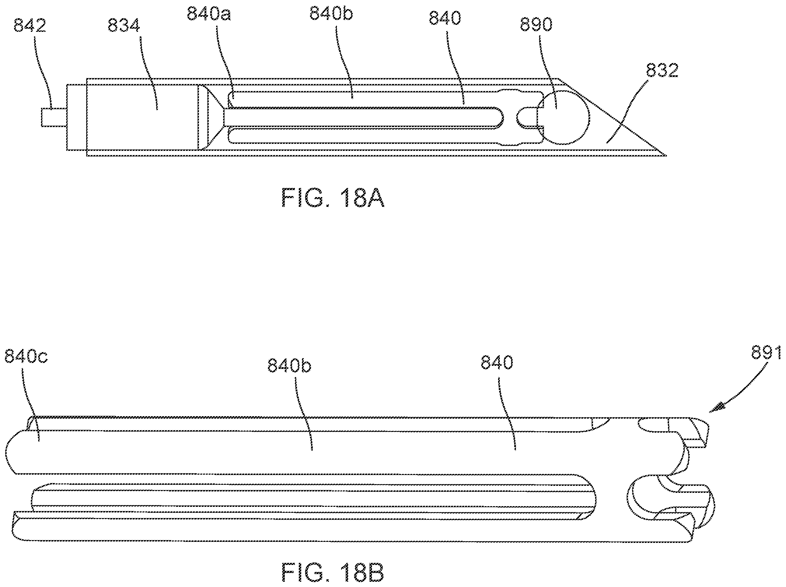

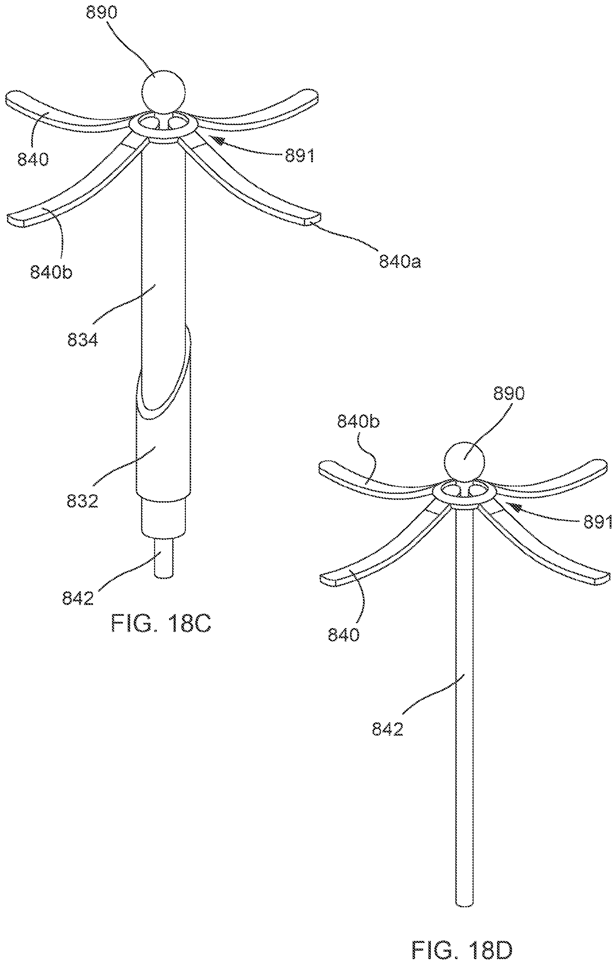

In an embodiment of a distal anchor of the present invention, the circumferential windings of the knot in the knot distal anchor 240 described above are replaced by an expandable distal anchor, as shown in FIGS. 18A-18D. In this embodiment, the distal anchor 840 includes elongate members 840b with free ends 840a and a stopper receiving section 891. FIG. 18A illustrates the distal anchor 840 in an elongated delivery configuration and disposed within a lumen defined by a distal end portion 832 of a delivery device such that the free ends 840a of elongate members 840b are disposed proximal to the stopper receiving section 891 of the distal anchor 840. FIG. 18B illustrates the distal anchor 840 in the elongated delivery configuration. FIGS. 18C and 18D illustrate the distal anchor 840 in a deployed configuration. In this embodiment, a distal end portion of the suture 842 includes a stopper 890 and the stopper receiving section 891 of the distal anchor 840 is configured to cooperatively mate with the stopper 890. During delivery of the distal anchor 840, the interior walls of the distal end portion 832 can retain the distal anchor 840 in its elongated delivery configuration when the distal anchor 840 is disposed within a lumen defined by the distal end portion 832, as shown in FIG. 18A. Similar to distal anchor 240 described above, the distal anchor 840 can be deployed in a similar manner using any of the delivery devices described in the '225 application. For example, the distal anchor 840 can be coupled to a suture 842 and removably coupled to or otherwise in operable contact with a pusher 834. The distal anchor 840 can be delivered in the elongated configuration (see e.g., FIG. 18A) and moved to the deployed configuration (see e.g., FIGS. 18B and 18C) by pulling the suture 842 proximally and/or moving the pusher 834 distally (see e.g., FIG. 18C). In this manner, the stopper 890 of the suture 842 can be moved into contact with the stopper receiving section 891, and the stopper 890 and the stopper receiving section 891 can collectively facilitate the transition of the distal anchor 840 from the elongated delivery configuration to the expanded deployed configuration.

In an example not forming part of the present invention, a distal anchor can be configured similar to the distal anchor 840 except that the distal anchor can be disposed on the suture 842 such that the free ends of the elongate members are distal to the stopper receiving section. In such an example, the distal anchor can be formed with for example a shape-memory material such that the distal anchor has a biased expanded or deployed configuration and an elongated collapsed configuration when constrained within a delivery device. The distal anchor can be pushed or moved out of a delivery device with, for example, a pusher device. As the distal anchor exits the delivery device, a distal end of the distal anchor can transition from its elongated collapsed configuration to its expanded, deployed or biased configuration. Said another way, as the distal anchor exits the distal end of the delivery device, the free ends of the elongate members can extend radially towards the deployed or biased configuration of the distal anchor. In this manner, the distal anchor can transition from its delivery configuration to its deployed configuration as it exits the delivery device.

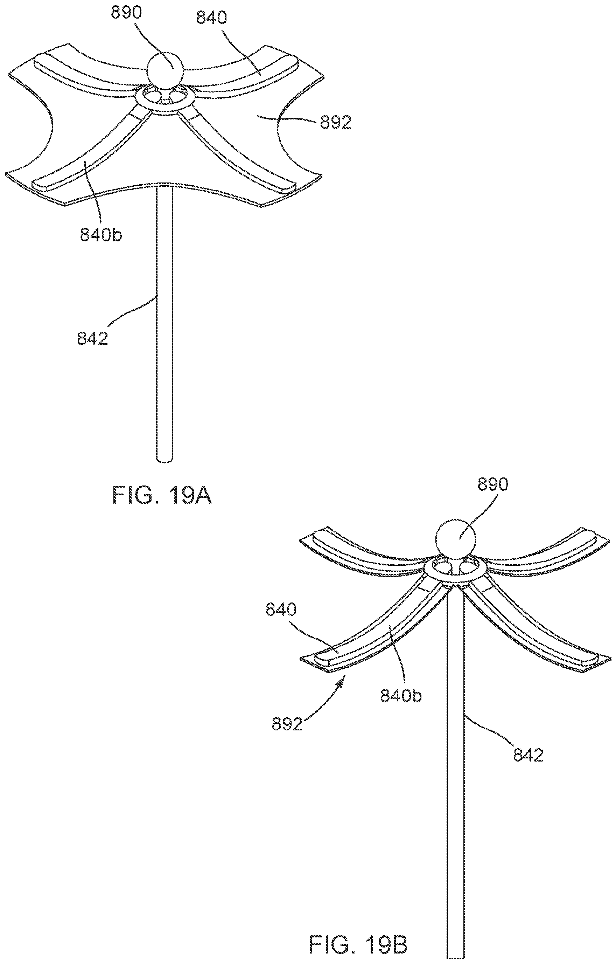

The distal anchor 840 can be formed of any suitable material, such as, for example a malleable stainless steel, a shape memory or superelastic alloy, or a polymer. One such polymer, for example, can include polyaryletherketones (PAEKs) such as polyetheretherketone (PEEK). Optionally, in some embodiments, a distal anchor can include or be coupled to a material (e.g., a fabric and/or polymer) that is configured to distribute an anchor load, cover and/or seal the hole made in the leaflet, and/or promote ingrowth or an otherwise desirable biological response when the distal anchor is disposed within a heart. For example, as illustrated in FIGS. 19A and 19B, the distal anchor 840 of FIGS. 18A-18D can have such a material 892 coupled thereto. For example, in some embodiments, the material 892 can extend between the elongate members 840b and beyond the free ends 840a of the distal anchor 840, as shown in FIG. 19A. In some embodiments, the material 892 can be sized and shaped to replicate or nearly replicate the size and shape of the elongate members 840b of the distal anchor 840, as shown in FIG. 19B.

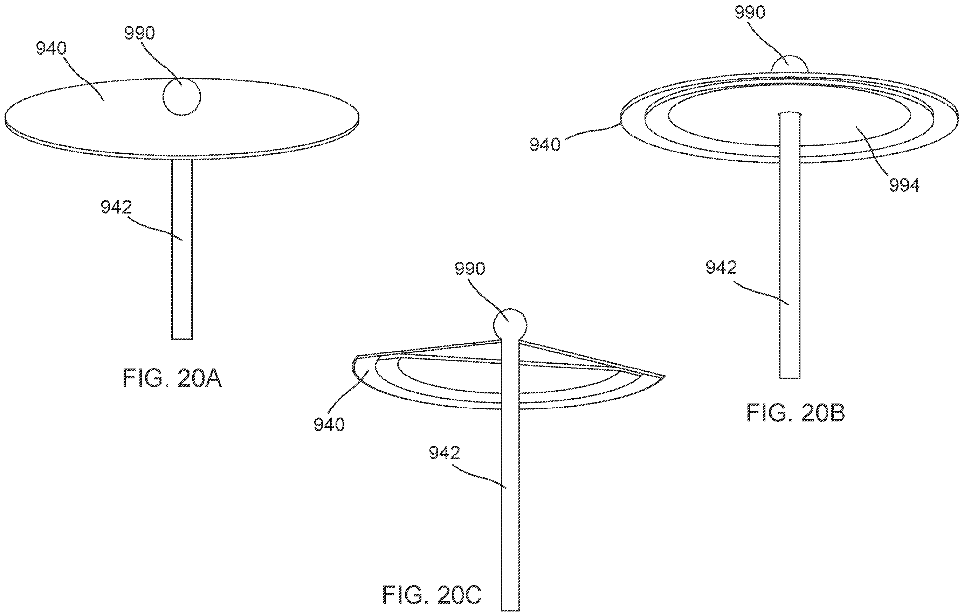

In another example of a distal anchor not forming part of the present invention, the circumferential windings of the knot in the knot distal anchor 240 described above are replaced by an expandable distal anchor, as shown in FIGS. 20A-20C. FIGS. 20A illustrates a distal end portion of the distal anchor 940 in a deployed configuration. FIG. 20B illustrates a proximal end portion of the distal anchor 940 in the deployed configuration. FIG. 20C illustrates in partial cross-section the distal anchor 940 in the deployed configuration. In this example, a distal end portion of the suture 942 includes a stopper 990. A radial support member 994 is coupled and disposed proximal to the proximal end portion of the distal anchor 940. The radial support member 994 can prevent or otherwise limit the distal anchor 940 from undesirably flipping or deflecting (1) beyond a plane defined by the stopper 990, and/or (2) distal to the stopper 990. The radial support member 994 can be made of any suitable material sufficient to provide radial support, such as, for example, a non-elastic material. In addition, as shown best in FIG. 20C, the distal anchor 940 is pre-configured to have a slight angle.

Similar to distal anchor 240 described above, the distal anchor 940 can be deployed in a similar manner using any of the delivery devices described in the '225 application. For example, the distal anchor 940 can be coupled to the suture 942 and removably coupled to or otherwise in operable contact with a pusher (not shown). The distal anchor 940 can be delivered in the elongated configuration (not shown) and moved to the deployed configuration by pulling the suture 942 proximally and/or moving the pusher (not shown) distally, as shown in FIGS. 20A-20C. In this manner, the stopper 990 of the suture 942 can be moved into contact with the distal end portion of the distal anchor 940, and as a result, can collectively facilitate the transition of the distal anchor 940 from the elongated delivery configuration (not shown) to the expanded deployed configuration. Although not shown, in some examples, the distal anchor 940 can include radial stiffening members in addition to or instead being coupled to the radial support member 994.

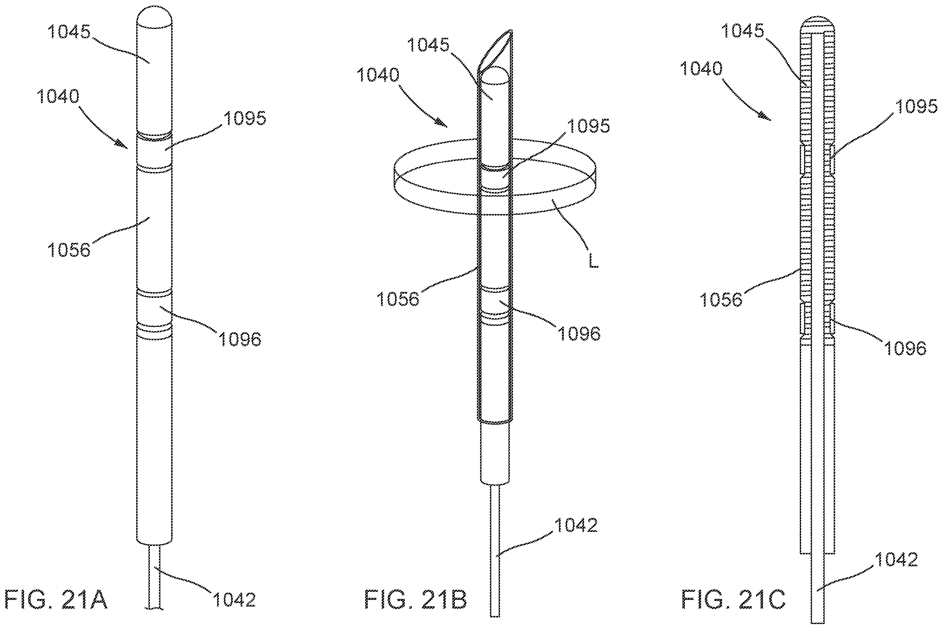

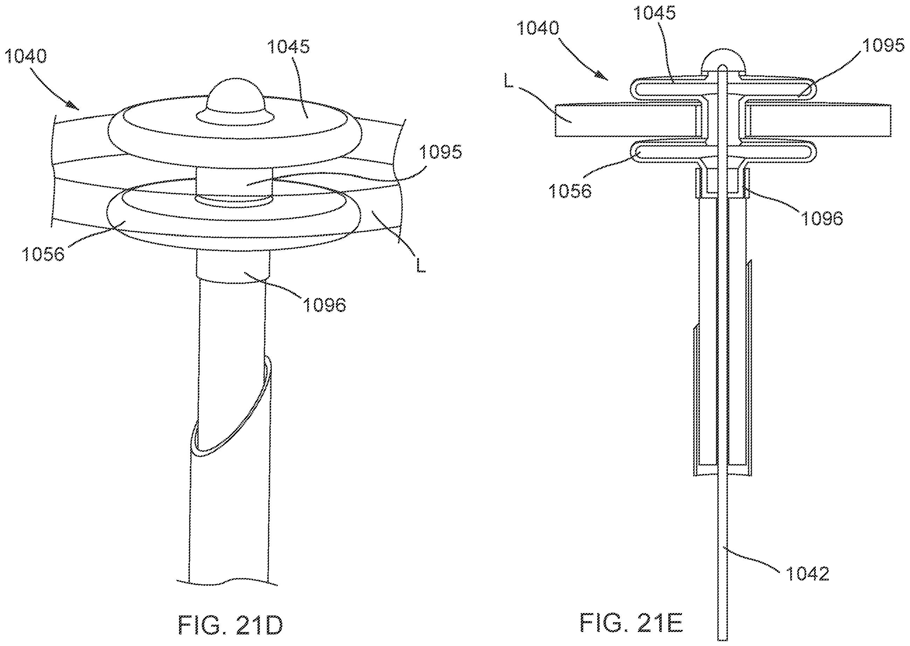

In another example of a distal anchor not forming part of the present invention, the circumferential windings of the knot in the knot distal anchor 240 described above are replaced by an expandable braid, as shown in FIGS. 21A-21E. FIG. 21A illustrates the expandable braid distal anchor 1040 in an elongated delivery configuration, FIG. 21B illustrates the distal anchor 1040 in the elongated delivery configuration with reference to a valve leaflet L, and FIG. 21C illustrates the distal anchor 1040 in cross-section in the elongated delivery configuration. FIG. 21D illustrates the distal anchor 1040 in an expanded or deployed configuration with reference to a valve leaflet L, and FIG. 21E illustrates in cross-section the distal anchor 1040 in the deployed configuration. In this example, the expandable braid distal anchor 1040 has a distal portion 1045, a proximal portion 1056, and a distal collar 1095 disposed therebetween. The distal anchor 1040 also includes a proximal collar 1096 disposed proximal to the proximal portion 1056 of the distal anchor 1040. Similar to the knot distal anchor 240 described above, the distal anchor 1040 can be deployed in a similar manner using any of the delivery devices described in the '225 application. For example, the distal anchor 1040 can be coupled to a suture 1042. The distal anchor 1040 can be delivered in the elongate configuration and moved to the deployed configuration by pulling the suture strands 1042 proximally to cause the braided distal portion 1045 and the braided proximal portion 1056 to expand radially, as shown in FIGS. 21D and 21E.

Prior to deployment of the expandable braid distal anchor 1040, the distal collar 1095 can be aligned with and disposed at least partially within the hole formed in the leaflet L, as shown in FIG. 21B. In this manner, when deployed (radially expanded), the distal portion 1045 of the distal anchor 1040 will be disposed on the distal side of the leaflet L (e.g., within the atrium of the heart), and the proximal portion 1046 of the distal anchor 1040 will be disposed on the proximal side of the leaflet L (e.g., within the ventricle of the heart), as shown in FIG. 21D. Deployment of the distal portion 1045 and the proximal portion 1046 can be initiated in stages. For example, deployment of the distal portion 1045 can be initiated while the proximal portion 1046 is in the elongated delivery configuration, and deployment of the proximal portion 1046 can be initiated after the distal portion 1045 has transitioned into the deployed configuration.

Further to this example, in use, the distal anchor 1040 can be inserted into the atrium of the heart and the distal portion 1045 can be deployed within the atrium. Next, the suture 1042 can be pulled proximally such that a proximal side surface of the distal portion 1045 of the distal anchor 1040 is brought into contact with an atrial side of the heart valve leaflet L. In this manner, the distal portion 1045 can be manipulated into a desirable position before the proximal portion 1046 of the distal anchor 1040 is deployed. Once the distal portion 1045 is suitable positioned against the valve leaflet L, the proximal portion 1046 of the distal anchor 1040 can be deployed such that a distal side surface of the proximal portion 1046 is brought into contact with a ventricle side of the valve leaflet L, thereby securing the leaflet L between the distal portion 1045 and the proximal portion 1046.

Although not shown, in some examples, the distal anchor 1040 can include a locking mechanism configured to lock, bias, or otherwise maintain the distal anchor 1040 in its expanded deployed configuration. Further, in some examples, the distal portion 1045 and the proximal portion 1046 can be formed of shape memory or superelastic material such that its expanded deployed configuration is its unbiased configuration.

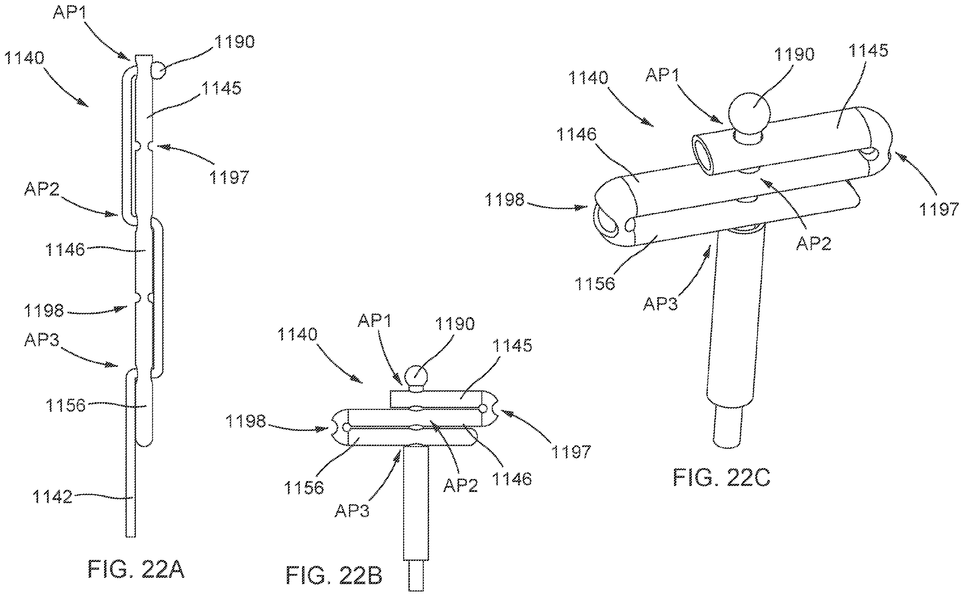

In another example of a distal anchor not forming part of the present invention, the circumferential windings of the knot in the knot distal anchor 240 described above are replaced by a single flexible tube, as shown in FIGS. 22A-22C. FIG. 22A illustrates the distal anchor 1140 in an elongated delivery configuration, and FIGS. 22B and 22C illustrate the distal anchor 1140 in a deployed configuration, in side view and perspective view, respectively. In this example, the flexible tube 1140 has a distal portion 1145, and a proximal portion 1156, and a medial portion 1146 disposed therebetween. Each portion is separated by a hinge section, i.e., a first hinge section 1197 is disposed between the distal portion 1145 and the medial portion 1146, and a second hinge section 1198 is disposed between the medial portion 1146 and the proximal portion 1156. The suture 1142 includes a stopper 1190 at its distal end, and extends therefrom through a first aperture AP1, a second aperture AP2 and a third aperture AP3, each of which is defined by the flexible tube 1140, as shown in FIGS. 22A-22C.

Similar to the knot distal anchor 240 described above, the distal anchor 1140 can be deployed in a similar manner using any of the delivery devices described in the '225 application. The distal anchor 1140 can be delivered in the elongate configuration and moved to the deployed configuration by pulling the suture strand 1142 proximally to deflect the portions 1145, 1146, 1156 about their respective hinge sections 1197, 1198, as shown in FIGS. 22B and 22C. In this manner, the portions 1145, 1146, 1156 are drawn towards each other (or folded onto one another) to form the expanded deployed configuration.

The distal anchor 1140 can be formed of any suitable material, e.g., ePFTE or a similar biocompatible polymer. In an alternative example, instead of a single flexible tube 1144, the anchor 1140 can be formed of separate portions and then coupled together. Further, in an alternative example, instead of three portions (i.e., distal, proximal, medial), the anchor 1140 can include any suitable number of portions (e.g., a single portion or four or more portions).

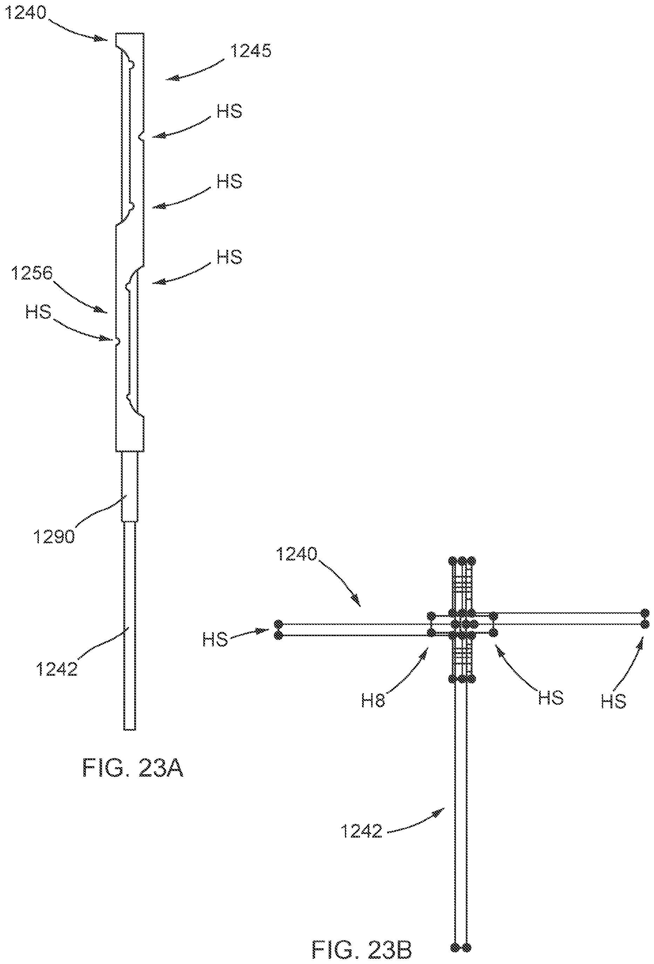

In another example of a distal anchor not forming part of the present invention, the circumferential windings of the knot in the knot distal anchor 240 described above are replaced by a hinged tube, as shown in FIGS. 23A and 23B. FIG. 23A illustrates the distal anchor 1240 in an elongated delivery configuration, and FIG. 23B illustrates schematically the distal anchor 1240 in a deployed configuration. In this example, the hinged tube 1240 has a distal portion 1245, and a proximal portion 1256, and hinge sections HS to facilitate deployment, deflection or bending of the distal portion 1245 and the proximal portion 1256 at desirable sections of the distal anchor 1240. A distal end portion of an elongated tube 1299 is fixedly coupled to a distal end of the distal portion 1245 of the distal anchor 1240, and extends through a lumen defined by the distal anchor 1240, out a proximal end of the proximal portion 1245 of the distal anchor 1240, and then coupled to a suture 1242, as shown in FIG. 23A. Similar to the knot distal anchor 240 described above, the distal anchor 1240 can be deployed in a similar manner using any of the delivery devices described in the '225 application. For example, the distal anchor 1240 can be delivered in the elongate configuration and moved to the deployed configuration by pulling the suture strand 1242 proximally, and thereby similarly moving the elongated tube 1299 proximally with the suture strand 1242, to deflect the hinged tube 1240 laterally with respect to the hinge sections HS to form the deployed or expanded configuration, as shown in FIG. 23B.

While some of the distal anchors described above as being delivered to a left ventricle of a heart, piercing a native mitral valve leaflet from the ventricular side to the atrial side, deploying the distal anchor on the atrial side of the leaflet, and anchoring the distal anchor to an apex region of the heart, in other instances, the distal anchors described above can be delivered and deployed via other suitable methods, e.g., transfemorally, transatrially and/or via an inferior vena cava (IVC). For example, one or more native valve leaflets can be pierced from the atrial side to the ventricular side, and the distal anchor can be delivered from the atrial side to the ventricular side and deployed in the ventricle. In such examples, in some instances, the distal anchor can be attached or otherwise coupled to (e.g., via a suture) a second distal anchor (e.g., deployed at a second leaflet). In some instances, the distal anchor can be anchored to the apical region of the heart by routing a suture attached to the anchor through the area or void between the leaflets from the atrial side to the ventricular side.

While various embodiments and examples useful for understanding the present invention have been described above, it should be understood that they have been presented by way of example only, and not limitation. Where methods described above indicate certain events occurring in certain order, the ordering of certain events may be modified. Additionally, certain of the events may be performed concurrently in a parallel process when possible, as well as performed sequentially as described above.

Where schematics and/or embodiments/examples described above indicate certain components arranged in certain orientations or positions, the arrangement of components may be modified. While the embodiments/examples have been particularly shown and described, it will be understood that various changes in form and details may be made. Any portion of the apparatus and/or methods described herein may be combined in any combination, except mutually exclusive combinations. The embodiments/examples described herein can include various combinations and/or sub-combinations of the functions, components and/or features of the different embodiments described.

## Claims

1. An apparatus, comprising:
a needle (832) having a bore and a distal tip suitable for penetrating a heart valve leaflet;
a pusher (834) having a distal end, the pusher (834) being disposed within the bore of the needle (832) for movement between a delivery position in which the distal end of the pusher (834) is proximate to the distal tip of the needle (832) and a deployed position in which the distal end of the pusher (834) is distal to the distal tip of the needle (832);
a distal anchor (840) having a distal end and a proximal end, the distal end having a stopper receiving portion (891), the distal anchor (840) having a plurality of elongate members (840b) extending proximally from the stopper receiving portion (891), each elongate member have a free proximal end (840a), the free proximal ends (840a) defining the proximal end of the distal anchor (840), the distal anchor (840) having a central bore extending through the stopper receiving portion (891) and between the plurality of elongate members (840b), the distal anchor (840) being disposed within the bore of the needle (832) in a delivery configuration in which the plurality of elongate members (840b) are oriented substantially axially and movable between a delivery position in which the distal anchor (840) is disposed between the distal end of the pusher (834) and the distal tip of the needle (832) and a deployed position in which the proximal end of the distal anchor (840) is disposed distal to the distal tip of the needle (832); and
an artificial chordae (842) having a proximal portion and a distal portion terminating in a stopper (890) and disposed within the bore of the distal anchor (840) with the stopper (890) distal to the stopper receiving portion (891) of the distal anchor (840) and the proximal portion proximal to the proximal end of the distal anchor (840),
the distal anchor (840) being reconfigurable from the delivery configuration to a deployed configuration when the distal anchor (840) is in the deployed position in which the plurality of elongate members (840b) are disposed with their free proximal ends (840a) spaced radially outwardly from the artificial chordae (842) a distance sufficient to anchor the artificial chordae (842) to the heart valve leaflet,
wherein the distal anchor (840) is reconfigurable from the delivery configuration to the deployed configuration by moving the distal end of the pusher (834) distally towards the distal end of the distal anchor (840) past the free proximal ends (840a) of, and between, the elongate members (840b) to urge the elongate members (840b) radially outwardly.

2. The apparatus of claim 1, further comprising a material (892) coupled to a proximal side of each of the elongate members (840b) and configured to promote tissue ingrowth with the heart valve leaflet.

3. The apparatus of claim 2, wherein the material (892) extends between the elongate members (892).

## Patentansprüche

1. Vorrichtung, aufweisend:
eine Nadel (832) mit einer Bohrung und einer distalen Spitze, die zum Penetrieren eines Klappensegels geeignet ist;
einen Schieber (834) mit einem distalen Ende, wobei der Schieber (834) in der Bohrung der Nadel (832) angeordnet ist zum Bewegen zwischen einer Zuführposition, in der das distale Ende des Schiebers (834) proximal zur distalen Spitze der Nadel (832) ist, und einer ausgebrachten Position, in der das distale Ende des Schiebers (834) distal zur distalen Spitze der Nadel (832) ist;
einen distalen Anker (840) mit einem distalen Ende und einem proximalen Ende, wobei das distale Ende einen Anschlagaufnahmeabschnitt (891) aufweist, wobei der distale Anker (840) eine Mehrzahl von länglichen Gliedern (840b) hat, die sich proximal von dem Anschlagaufnahmeabschnitt (891) erstrecken, wobei jedes länglichen Glieder ein freies proximales Ende (840a) hat, wobei die freien proximalen Enden (840a) das proximale Ende des distalen Ankers (840) definieren, wobei der distale Anker (840) eine zentrale Bohrung aufweist, die sich durch den Anschlagaufnahmeabschnitt (891) und zwischen der Mehrzahl von länglichen Gliedern (840b) erstreckt, wobei der distale Anker (840) in der Bohrung der Nadel (832) in einer Zuführkonfiguration, in der die Mehrzahl von länglichen Gliedern (840b) im Wesentlichen axial ausgerichtet sind, angeordnet ist und zwischen einer Zuführposition, in der der distale Anker (840) zwischen dem distalen Ende des Schiebers (834) und der distalen Spitze der Nadel (832) angeordnet ist, und einer ausgebrachten Position, in der das proximale Ende des distalen Ankers (840) distal zur distalen Spitze der Nadel (832) angeordnet ist, bewegbar ist; und
eine künstliche Chordae (842), die einen proximalen Abschnitt und einem distalen Abschnitt, der in einem Anschlag (890) endet, hat und die innerhalb der Bohrung des distalen Ankers (840) angeordnet ist mit dem Anschlag (890) distal zum Anschlagaufnahmeabschnitt (891) des distalen Ankers (840) und dem proximalen Abschnitt proximal zum proximalen Ende des distalen Ankers (840),
wobei der distale Anker (840) von der Zuführkonfiguration in eine ausgebrachte Konfiguration rekonfigurierbar ist, wenn der distale Anker (840) in der entfalteten Position ist, in der die Mehrzahl von länglichen Gliedern (840b) angeordnet sind mit ihren freien proximalen Enden (840a) radial nach außen von den künstlichen Chordae (842) um einen Abstand beabstandet, der ausreicht, um die künstlichen Chordae (842) an dem Klappensegel zu verankern,
wobei der distale Anker (840) durch distales Bewegen des distalen Endes des Schiebers (834) in Richtung des distalen Endes des distalen Ankers (840) vorbei an und zwischen den freien proximalen Enden (840a) der langgestreckten Glieder (840b), um die langgestreckten Elemente (840b) radial nach außen zu drücken, von der Zuführkonfiguration zur ausgebrachten Konfiguration rekonfigurierbar ist.

2. Vorrichtung nach Anspruch 1, ferner aufweisend ein Material (892), das mit einer proximalen Seite jedes der länglichen Glieder (840b) verbunden und dazu ausgebildet ist, das Einwachsen von Gewebe in das Klappensegel zu fördern.

3. Vorrichtung nach Anspruch 2, wobei sich das Material (892) zwischen den länglichen Gliedern (892) erstreckt.

## Revendications

1. Appareil comprenant :
une aiguille (832) présentant un trou et une pointe distale appropriée pour pénétrer dans un feuillet de valvule cardiaque ;
un poussoir (834) présentant une extrémité distale, le poussoir (834) étant disposé à l'intérieur du trou de l'aiguille (832) pour un mouvement entre une position de pose, dans laquelle l'extrémité distale du poussoir (834) est à proximité de la pointe distale de l'aiguille (832), et une position déployée dans laquelle l'extrémité distale du poussoir (834) est distale par rapport à la pointe distale de l'aiguille (832) ;
un ancrage distal (840) présentant une extrémité distale et une extrémité proximale, l'extrémité distale présentant une partie de réception de bouchon (891), l'ancrage distal (840) présentant une pluralité d'éléments allongés (840b) s'étendant de manière proximale à partir de la partie de réception de bouchon (891), chaque élément allongé présentant une extrémité proximale libre (840a), les extrémités proximales libres (840a) définissant l'extrémité proximale de l'ancrage distal (840), l'ancrage distal (840) présentant un trou central s'étendant à travers la partie de réception de bouchon (891) et entre la pluralité d'éléments allongés (840b), l'ancrage distal (840) étant disposé à l'intérieur du trou de l'aiguille (832) dans une configuration de pose, dans laquelle la pluralité d'éléments allongés (840b) sont orientés sensiblement axialement, et étant mobile entre une position de pose, dans laquelle l'ancrage distal (840) est disposé entre l'extrémité distale du poussoir (834) et la pointe distale de l'aiguille (832), et une position déployée, dans laquelle l'extrémité proximale de l'ancrage distal (840) est disposée de manière distale par rapport à la pointe distale de l'aiguille (832) ; et
un cordage artificiel (842) présentant une partie proximale et une partie distale se terminant par un bouchon (890) et disposé à l'intérieur du trou de l'ancrage distal (840), le bouchon (890) étant distal par rapport à la partie de réception de bouchon (891) de l'ancrage distal (840) et la partie proximale étant proximale par rapport à l'extrémité proximale de l'ancrage distal (840),
l'ancrage distal (840) étant reconfigurable à partir de la configuration de pose vers une configuration déployée lorsque l'ancrage distal (840) est dans la position déployée, dans laquelle la pluralité d'éléments allongés (840b) sont disposés avec leurs extrémités proximales libres (840a) écartées radialement vers l'extérieur à partir du cordage artificiel (842) d'une distance suffisante pour ancrer le cordage artificiel (842) au feuillet de valvule cardiaque,
l'ancrage distal (840) étant reconfigurable à partir de la configuration de pose vers la configuration déployée par déplacement de l'extrémité distale du poussoir (834) de manière distale vers l'extrémité distale de l'ancrage distal (840) au-delà des extrémités proximales libres (840a) des éléments allongés (840b) est entre ceux-ci pour pousser les éléments allongés (840b) radialement vers l'extérieur.

2. Appareil selon la revendication 1, comprenant en outre un matériau (892) accouplé à un côté proximal de chacun des éléments allongés (840b) et conçu pour favoriser une interposition tissulaire avec le feuillet de valvule cardiaque.

3. Appareil selon la revendication 2, le matériau (892) s'étendant entre les éléments allongés (892).
